(19) Europäisches Patentamt
European Patent Office
Office européen des brevets

(11) **EP 3 867 914 B1**

(12) **EUROPÄISCHE PATENTSCHRIFT**

(45) Veröffentlichungstag und Bekanntmachung des Hinweises auf die Patenterteilung:
**28.01.2026 Patentblatt 2026/05**

(21) Anmeldenummer: **19784113.3**

(22) Anmeldetag: **16.10.2019**

(51) Internationale Patentklassifikation (IPC):
**G16C 20/10** (2019.01)    **G16C 10/00** (2019.01)

(52) Gemeinsame Patentklassifikation (CPC):
**G16C 20/10;** G16C 10/00

(86) Internationale Anmeldenummer:
**PCT/EP2019/078115**

(87) Internationale Veröffentlichungsnummer:
**WO 2020/079094 (23.04.2020 Gazette 2020/17)**

(54) **MONTE-CARLO-METHODE ZUR AUTOMATISIERTEN UND HOCH-EFFIZIENTEN BERECHNUNG VON KINETISCHEN DATEN CHEMISCHER REAKTIONEN**

MONTE CARLO METHOD FOR THE AUTOMATED AND HIGHLY EFFICIENT CALCULATION OF KINETIC DATA OF CHEMICAL REACTIONS

MÉTHODE DE MONTE CARLO POUR LE CALCUL AUTOMATISÉ ET HAUTEMENT EFFICACE DE DONNÉES CINÉTIQUES DE RÉACTIONS CHIMIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.10.2018 EP 18201344**

(43) Veröffentlichungstag der Anmeldung:
**25.08.2021 Patentblatt 2021/34**

(73) Patentinhaber: **Covestro Deutschland AG**
**51373 Leverkusen (DE)**

(72) Erfinder:
• **LEVEN, Matthias**
**51069 Köln (DE)**
• **GAMEZ, Jose**
**51063 Köln (DE)**
• **REHM, Kunibert**
**51377 Leverkusen (DE)**

(74) Vertreter: **Levpat**
**c/o Covestro AG**
**Gebäude 4825**
**51365 Leverkusen (DE)**

(56) Entgegenhaltungen:
EP-A1- 2 352 107    WO-A1-2018/102565
CN-A- 104 765 918    JP-A- 2008 250 392
JP-A- H1 125 063    US-A1- 2003 236 655

• LIN X.-X. ET AL: "A flexible transition state searching method for atmospheric reaction systems", CHEMICAL PHYSICS, NORTH-HOLLAND, NL, vol. 450, 11 February 2015 (2015-02-11), pages 21 - 31, XP029204426, ISSN: 0301-0104, DOI: 10.1016/ J.CHEMPHYS.2015.02.002
• SWIHART M. T. ET AL: "Assembling gas-phase reaction mechanisms for high temperature inorganic systems based on quantum chemistry calculations and reaction rate theories", JOURNAL OF PHYSICS AND CHEMISTRY OF SOLIDS, PERGAMON PRESS, LONDON, GB, vol. 66, no. 2-4, 1 February 2005 (2005-02-01), pages 364 - 371, XP027709615, ISSN: 0022-3697, [retrieved on 20050201]
• HAFNER J.: "Adsorption and reaction of organic molecules on solid surfaces - ab-initio density functional investigations", MONATSHEFTE FÜR CHEMIE - CHEMICAL MONTHLY ; AN INTERNATIONAL JOURNAL OF CHEMISTRY, SPRINGER-VERLAG, AU, vol. 139, no. 4, 14 March 2008 (2008-03-14), pages 373 - 387, XP019592602, ISSN: 1434-4475

- MARTÍNEZ-NÚÑEZ E.: "An automated transition state search using classical trajectories initialized at multiple minima", PHYSICAL CHEMISTRY CHEMICAL PHYSICS, vol. 17, no. 22, 6 May 2015 (2015-05-06), pages 14912 - 14921, XP055601422, ISSN: 1463-9076, DOI: 10.1039/C5CP02175H
- RODRÍGUEZ A. ET AL: "tsscds2018: A code for automated discovery of chemical reaction mechanisms and solving the kinetics", JOURNAL OF COMPUTATIONAL CHEMISTRY., vol. 39, no. 23, 5 September 2018 (2018-09-05), GB, pages 1922 - 1930, XP055601919, ISSN: 0192-8651, DOI: 10.1002/jcc.25370
- JACOBSON L. D. ET AL: "Automated Transition State Search and Its Application to Diverse Types of Organic Reactions", JOURNAL OF CHEMICAL THEORY AND COMPUTATION: JCTC, vol. 13, no. 11, 17 October 2017 (2017-10-17), US, pages 5780 - 5797, XP055601412, ISSN: 1549-9618, DOI: 10.1021/acs.jctc.7b00764
- SALCICCIOLI M. ET AL: "A review of multiscale modeling of metal-catalyzed reactions: Mechanism development for complexity and emergent behavior", CHEMICAL ENGINEERING SCIENCE, OXFORD, GB, vol. 66, no. 19, 30 May 2011 (2011-05-30), pages 4319 - 4355, XP028264661, ISSN: 0009-2509, [retrieved on 20110613], DOI: 10.1016/J.CES.2011.05.050
- KEIL F. J.: "Complexities in modeling of heterogeneous catalytic reactions", COMPUTERS & MATHEMATICS WITH APPLICATIONS, ELSEVIER, AMSTERDAM, NL, vol. 65, no. 10, 3 January 2013 (2013-01-03), pages 1674 - 1697, XP028556589, ISSN: 0898-1221, DOI: 10.1016/J.CAMWA.2012.11.023
- DIEDRICH M. K. ET AL: "Experimental Determination of the Activation Parameters and Stereoselectivities of the Intramolecular Diels-Alder Reactions of 1,3,8-Nonatriene, 1,3,9-Decatriene, and 1,3,10-Undecatriene and Transition State Modeling with the Monte Carlo-Jumping Between Wells/Molecular Dynamics Method", JOURNAL OF THE AMERICAN CHEMICAL SOCIETY, vol. 119, no. 43, 1 October 1997 (1997-10-01), pages 10255 - 10259, XP055593729, ISSN: 0002-7863, DOI: 10.1021/ja9643331
- MERCERO J. M. ET AL: "Theoretical methods that help understanding the structure and reactivity of gas phase ions", INTERNATIONAL JOURNAL OF MASS SPECTROMETRY, ELSEVIER SCIENCE PUBLISHERS, AMSTERDAM, NL, vol. 240, no. 1, 1 January 2005 (2005-01-01), pages 37 - 99, XP027705154, ISSN: 1387-3806, [retrieved on 20050101]
- KRATZER P.: "Monte Carlo and kinetic Monte Carlo methods", ARXIV.ORG, CORNELL UNIVERSITY LIBRARY, 201 OLIN LIBRARY CORNELL UNIVERSITY ITHACA, NY 14853, 16 April 2009 (2009-04-16), XP080320087
- GREBNER C. ET AL: "PathOpt-A global transition state search approach: Outline of algorithm", JOURNAL OF COMPUTATIONAL CHEMISTRY., vol. 34, no. 21, 4 May 2013 (2013-05-04), GB, pages 1810 - 1818, XP055601427, ISSN: 0192-8651, DOI: 10.1002/jcc.23307
- LIN Y. ET AL: "Reliable Modeling and Optimization for Chemical Engineering Applications: Interval Analysis Approach", RELIABLE COMPUTING ; AN INTERNATIONAL JOURNAL DEVOTED TO RELIABLE MATHEMATICAL COMPUTATIONS BASED ON FINITE REPRESENTATIONS AND GUARANTEED ACCURACY, KLUWER ACADEMIC PUBLISHERS, DO, vol. 12, no. 6, 25 October 2006 (2006-10-25), pages 427 - 450, XP019453859, ISSN: 1573-1340, DOI: 10.1007/S11155-006-9013-6

**Beschreibung**

Die vorliegende Erfindung betrifft ein computerimplementiertes Verfahren zur Berechnung von Übergangszuständen einer chemischen Reaktion, sowie ein System zur Datenverarbeitung umfassend Mittel zur Ausführung des Verfahrens, ein Computerprogramm umfassend Befehle die einen Computer veranlassen das Verfahren durchzuführen und die Verwendung des Computerprogramms. Die Erfindung betrifft außerdem ein System, ein Verfahren und ein Mittel für die automatisierte und effiziente Bestimmung von kinetischen Daten chemischer Reaktionen.

Während einer chemischen Reaktion verändern die beteiligten Atome ihre Geometrie und Bindungen werden gebrochen und neue Bindungen gebildet. Dabei verändert sich auch die Energie der beteiligten Atome und erreicht während des Verlaufs der chemischen Reaktion einen Zustand maximaler Energie, den sogenannten Übergangszustand. Der Übergangszustand stellt einen Potentialwall oder eine Aktivierungsbarriere dar, der die Edukte von den Produkten der chemischen Reaktion trennt. Ist die Aktivierungsbarriere überwunden, so wird das Produkt gebildet. Die Energie während einer chemischen Reaktion kann mit Hilfe einer sogenannten Potentialhyperfläche dargestellt werden, welche die potentielle Energie der an der Reaktion beteiligten Atome in Abhängigkeit von ihrer Geometrie abbildet. Dabei bildet die Potentialhyperfläche auch Zustände ab, bei denen keine Produkte gebildet werden. Der direkte Reaktionspfad, bei dem Produkte unter Überwindung des Übergangszustandes gebildet werden, kann als Kurve gezeigt werden, bei der die Abstände der einzelnen Atome der Moleküle gegen die Energie aufgetragen werden. Mit Hilfe von quantenchemischen Methoden und mathematischen Näherungsverfahren kann die Energie der Geometrie eines Moleküls oder eines Systems von mehreren Molekülen berechnet werden, wobei der dabei zu betrachtende Funktionsraum die Freiheitsgrade des Moleküls umfasst. Diese Verfahren ermöglichen die Ermittlung der Geometrie am Übergangszustand und erlauben Vorhersagen über die Reaktionskinetik einer chemischen Reaktion.

Eine bekannte Methode mit der die Geometrie am Übergangszustand ermittelt werden kann, sind Newton-Raphson-Verfahren. Die Berechnung von Übergangszuständen mit Hilfe von Newton-Raphson-Verfahren weist jedoch einige Nachteile auf. Zunächst kann ein Newton-Raphson Algorithmus nicht auf jede beliebige Ausgangsgeometrie für ein Molekül angewendet werden, sondern nur auf Molekülgeometrien, die bereits sehr nah an die Geometrie des Übergangszustandes angenähert sind. Dies ist aufwendig, weil die Annäherung einer Molekülgeometrie an die Geometrie des Übergangszustandes üblicherweise händisch erfolgt, d.h. es werden Bindungslängen per Hand am Computer eingestellt. Es sind auch Methoden bekannt, bei denen zunächst mit anderen Methoden Potentialhyperflächen berechnet werden aus denen mögliche Sattelpunkte identifiziert werden. Auch bei diesen Methoden muss also erst eine Geometrie ermittelt werden, die bereits sehr nah an die Geometrie des Übergangszustandes angenähert ist. Aus diesen Gründen ist die Berechnung von Übergangszuständen mit Hilfe von Newton-Raphson-Algorithmen insbesondere für Moleküle mit mehr als 100 Atomen mit der derzeitigen Computertechnik besonders zeit- und kostenintensiv. Zur Anwendung von Newton Raphson Prozeduren und darauf aufbauenden Näherungen müssen außerdem zwingend zweite Ableitungen der Energie nach den Kernkoordinaten des betrachteten Moleküls berechnet werden. Der Rechenaufwand der zweiten Ableitungen skaliert quadratisch mit der Größe des Moleküls und ist deswegen für die Berechnung von Aktivierungsenergien geschwindigkeitsbestimmend.

Lin et al. ("A flexible transition state searching method for atmospheric reaction systems," Chemical Physics 450-451, 2015, S. 21-31) offenbaren eine Methode zur Untersuchung atmosphärischer chemischer Reaktionen in der Gasphase. Die Methode umfasst in einem ersten Schritt ein auf Monte-Carlo-Methoden basiertes Screening von Potentialhyperflächen mit Kraftfeld-Methoden, wobei genäherte Sattelpunkt-artige Regionen identifiziert werden. Die verwendete Observable der entsprechenden Monte Carlo Methode ist dabei der Wert einer Energiefunktion. Darauf aufbauend wird dann versucht mittels Newton-Raphson Methodenchemische Übergangszustände zu lokalisieren. Mit der hier offenbarten Monte-Carlo-Methode muss zunächst die gesamte Potentialhyperfläche simuliert werden, bevor überhaupt genäherte Sattelpunkt-artige Regionen identifiziert werden können. Folglich können mit dieser Monte-Carlo-Methode nicht gezielt Sattelpunkt-artige Regionen optimiert werden. Aus diesem Grund ist keine effiziente Nutzung der verwendeten Rechenressourcen zur Lokalisierung der benötigten Sattelpunkte möglich. Der Einsatz der hier offenbarten Methode beschränkt sich mit der derzeitigen Computertechnik auf kleine Moleküle mit 7 bis 30 Atomen. Zudem werden mathematisch stark vereinfachte Methoden verwendet.

E. Martínez-Núñez et al. "An automated transition state search using classical trajectories initialized at multiple minima", Phys Chem Chem Phys, 14. Juni 2015, 17(22):14912-21; "tsscds2018: A code for automated discovery of chemical reaction mechanisms and solving the kinetics", J. Comp.Chem., 24. September 2018, 39(23)1922-1930) offenbaren eine Methode zur Untersuchung chemische Reaktionspfade. Dabei werden Potentialhyperflächen chemischer Moleküle mit mathematisch vereinfachten Methoden berechnet. Anschließend wird versucht die chemisch relevanten Übergangszustände mit gewöhnlichen Pseudo-Newton-Raphson-Methoden zu berechnen. Auch hier ist nachteilig, dass zunächst die gesamte Potentialhyperfläche berechnet werden muss, was zeitaufwändig ist und Rechnerleistung braucht. Zudem kann auch diese Methode nur auf kleinere Moleküle angewendet werden.

Jacobson et al. ("Automated Transition State Search and Its Application to Diverse Types of Organic Reactions", J. Chem. Theory Comput. 13, 11, 5780-5797) offenbaren eine Methode zur automatisierten Berechnung von chemischen Übergangszuständen. Dabei werden chemische Übergangszustände aus chemischen Gleichgewichtszuständen berechnet. Dieses Vorgehen benötigt eine separate Berechnung von chemischen Gleichgewichtszuständen, bevor eine Interpolation der erhaltenen Geometrien durchgeführt werden kann, mit welchen der Übergangszustand angenähert wird. In einem weiteren Schritt wird dann basierend auf dieser Annäherung versucht eine Übergangszustandsgeometrie zu berechnen. Das Erstellen der benötigten Gleichgewichtszustände erfordert zusätzlichen Arbeitsaufwand durch den Fachmann. Die in der offenbarten Methode verwendete Interpolation lässt sich nicht mit Erfolg auf beliebige Molekülgeometrien anwenden.

Hu et al. ("A gradient-directed Monte Carlo method for global optimization in a discrete space: Application to protein sequence design and folding" J Chem Phys. 2009 Oct 21; 131(15): 154117) offenbaren eine Methode zur Berechnung von Proteinstrukturen. Bei dieser Methode werden Faltungen von Proteinstrukturen mittels Monte-Carlo-Methoden berechnet. Zur Beschleunigung der Konvergenz der Monte-Carlo-Prozeduren werden Gradienten berechnet, welche in die Richtung und Größe von Auslenkungen der Atompositionen eingehen. Die Publikation lässt keine mögliche Verwendung dieser Methode zur gezielten Berechnung von Sattelpunkten erkennen.

Es besteht somit ein Bedarf für ein Verfahren für die Berechnung von Übergangszuständen, bei dem eine Molekülgeometrie für einen Übergangszustand einer chemischen Reaktion mit einem einfachen Verfahren, insbesondere einem computerimplementierten Verfahren, genähert werden kann. Insbesondere besteht ein Bedarf für ein Verfahren mit dem Übergangszustände ermittelt werden können, ohne zuvor eine Potentialhyperfläche berechnen zu müssen. Hierbei werden Verfahren verwendet die großen Rechenaufwand mit sich bringen und qualitativ weniger genau sind. Insbesondere soll möglichst auf die Anwendung von Verfahren verzichtet werden, die die Berechnung zweiter Ableitungen der Energie nach den Atomkoordinaten beinhalten, wie Newton Raphson oder pseudo Newton Raphson-Verfahren. Diese liefern entweder schlechte Erfolgsquoten beim Lokalisieren von Übergangs-zuständen oder sind besonders für große Moleküle von prohibitiver Ressourcenbeanspruchung . Auf diese Weise können Auslastungen von Ressourcen wie Prozessoren und Speichermedien reduziert werden.

Aufgabe der vorliegenden Erfindung ist es, ein computerimplementiertes Verfahren zur Verfügung zu stellen, bei dem eine Geometrie eines Übergangszustandes mit einem leicht anwendbaren, insbesondere einem computerimplementierten, Verfahren unter zur Hilfenahme einer quantenchemischen Methode berechnet wird. Dabei soll bevorzugt möglichst kein quantenchemisches Verfahren verwendet werden, bei dem die zweite Ableitung der Energie nach den Kernkoordinaten des betrachteten Moleküls berechnet werden muss, um den Übergangszustand berechnen zu können. Insbesondere soll eine Molekülgeometrie für einen Übergangszustand einer chemischen Reaktion ermittelt werden können, ohne zuvor die Potentialhyperfläche der chemischen Reaktion zu berechnen. Insbesondere soll ein computerimplementiertes Verfahren zur Berechnung des Übergangszustands für Moleküle mit bevorzugt mehr als 100 Atomen zur Verfügung gestellt werden.

Diese Aufgabe wurde gelöst durch ein computerimplementiertes Verfahren zur Berechnung von Übergangszuständen einer chemischen Reaktion umfassend die Schritte

### A Erzeugen einer Startgeometrie

A1 Bereitstellung der dreidimensionalen Darstellung mindestens eines Moleküls im energetischen Grundzustand,

A2 Auswahl von mindestens einer Bindung des mindestens einen Moleküls und Auswahl einer Länge der Bindung, wobei die ausgewählte Länge nicht der Länge der Bindung im energetischen Grundzustand des Moleküls entspricht, so dass eine Startgeometrie für die chemische Reaktion erhalten wird,

A3 Dreidimensionale Darstellung der Startgeometrie in kartesischen und/oder internen Koordinaten,

### B Ermittlung einer optimierten Startgeometrie

B1 Festlegung eines Funktionsraumes welcher die mindestens eine Bindung aus Schritt A2 und die durch diese Bindung verbundenen Atome umfasst,

B2 Geometrieoptimierung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion $E = f(x)$ der quantenchemischen Methode und erhalten wird, mit $E$=Gesamtenergie der optimierten Startgeometrie und $x$=Kernkoordinaten des Moleküls in der optimierten Startgeometrie,

B4.1 wenn die Gradient-Norm B3 $\nabla$ $0 \leq \nabla \leq 0{,}03$ $E_h\,a_0^{-1}$ beträgt, dann Einordnung der optimierten Startgeometrie als eine Vorstufe für den Übergangszustand der chemischen Reaktion und Fortführung des Verfahrens mit Schritt D1, oder

B4.2 wenn die Gradient-Norm B3 $\nabla > 0{,}03$ $E_h\,a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

**C Ermittlung der Vorstufe für den Übergangszustand der chemischen Reaktion**

C1 Variation der optimierten Startgeometrie unter Anwendung eines Monte-Carlo-Algorithmus wobei

C1.1 wenigstens ein Atom aus dem in Schritt B1 ausgewählten Funktionsraum zufällig ausgewählt wird,

C1.2 ein Vektor für eine Auslenkung des in Schritt C1 gewählten Atoms zufällig ausgewählt wird, wobei der zufällig ausgewählte Betrag des Vektors mit der Gradient-Norm B3 gewichtet wird,

C1.3 das Schritt C1.1 ausgewählte Atom anhand des Vektors aus Schritt C1.2 aus der Position des Atoms in der optimierten Startgeometrie ausgelenkt wird, so dass eine Vorstufe für einen Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schritt A2 die Länge hat, die in Schritt C3 ermittelt wurde,

C3 Ermittlung der Gradient-Norm C3 für die Vorstufe für den Übergangszustand aus Schritt C2, wobei die Gradient-Norm durch die erste Ableitung der Funktion E = f(x) der quantenchemischen Methode und erhalten wird, mit E=Gesamtenergie der Vorstufe für den Übergangszustand und x=Kernkoordinaten des Moleküls in der Vorstufe für den Übergangszustand,

C4.1 wenn die Gradient-Norm C3 $\nabla$ $0 \leq \nabla \leq 0{,}03$ $E_h$ $a_0^{-1}$ beträgt, dann Fortführung des Verfahrens mit Schritt D1, oder

C4.2 wenn die Gradient-Norm C3 $\nabla$ $> 0{,}03$ $E_h$ $a_0^{-1}$ $E_h$ $a_0^{-1}$ beträgt, dann Wiederholung der Schritte C1 bis C3 bis eine Gradient-Norm C3 $\nabla$ $0 \leq \nabla \leq 0{,}03$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Variation der Vorstufe aus Schritt C4.1 oder der Vorstufe aus Schritt B4.1, indem folgende Schritte für ein Atom innerhalb des in B1 definierten Funktionsraums durchgeführt werden

D1.1 Auswahl eines Atoms aus dem in Schritt B1 ausgewählten Funktionsraum,

D1.2 Auswahl eines Vektors für eine Auslenkung des in Schritt D1.1 gewählten Atoms,

D1.3 Auslenkung des in Schritt D1.1 ausgewählten Atoms anhand des Vektors aus Schritt D1.2 aus seiner Position in der Vorstufe, wobei die Position einmal um einen positiven Wert des Betrags des Vektors und einmal um einen negativen Wert des Betrags des Vektors ausgelenkt wird, so dass bei der Durchführung der Schritte D1.1 bis D.1.3 zwei ausgelenkte Vorstufen erhalten werden,

D2 Geometrieoptimierung der zwei ausgelenkten Vorstufen aus Schritt D1 mittels der quantenchemischen Methode unter der Randbedingung dass die in D1 erhaltenen Bindungsabstände konstant gehalten werden, so dass zwei optimierte Vorstufen (i) und (ii) erhalten werden,

D3 Berechnung der Energie der zwei optimierten Vorstufen (i) und (ii) aus Schritt D2 mittels des quantenchemischen Verfahrens,

D4 Vergleich des Wertes der Energie der zwei optimierten Vorstufen mit dem Wert der Gradient-Norm C3 oder B3 der Vorstufe, die in Schritt D1 verwendet wurde,

D4.1. wenn der Wert der Energie der optimierten Vorstufe (i) und der Wert der Energie der optimierten Vorstufe (ii) jeweils kleiner sind als der Wert der Energie C3 oder B3 der Vorstufe, die in Schritt D1 verwendet wurde, dann Einordnung der Vorstufe, die in Schritt D1 verwendet wurde, als Übergangszustand,

D4.2. wenn der Wert der Energie der optimierten Vorstufe (i) oder der Wert der Energie der optimierten Vorstufe (ii) nicht jeweils kleiner ist als der Wert der Energie C3 oder B3 der Vorstufe, die in Schritt D1 verwendet wurde, dann Wiederholung des Verfahrens ab Schritt C1.

Es wurde überraschend gefunden, dass die Aufgabe gelöst werden kann, indem durch Einsatz einer geeigneten Monte-Carlo-Methode verschiedene Molekülgeometrien variiert werden und mit Hilfe eines Gütekriteriums eine Geometrie für einen Übergangszustand angenähert wird. Des Weiteren wurde überraschend gefunden, dass die Aufgabe gelöst werden kann, indem bei dem Monte-Carlo-verfahren als Observable der Gradient der Kurve des direkten Reaktionspfades verwendet wird. Dadurch lässt sich der zu behandelnde Funktionsraum, der die Molekülgeometrie am Übergangszustand der chemischen Reaktion abbilden soll, derart reduzieren, dass die Berechnung von Sattelpunkten ermöglicht wird, ohne zuvor eine Potentialhyperfläche zu berechnen oder manuell Molekülgeometrien zu suchen. Außerdem wurde überraschend gefunden, dass mit dem erfindungsgemäßen Verfahren die Berechnung von Sattelpunkten in hochdimensionalen Funktionsräumen möglich wird ohne Newton-Raphsonbasierte Methoden zu verwenden. Statt der Berechnung von zweiten Ableitungen der Energie nach den Kernkoordinaten wird der Bereich um den Sattelpunkt durch infinitesimale Auslenkungen und anschließende Geometrieoptimierung verifiziert. Dabei muss die Auslenkung entlang der Bindungsdissoziation eine Abnahme der Gesamtenergie ergeben. Auf diese Weise kann die aufwendige Berechnung der zweiten Ableitungen der Energie nach den Kernkoordinaten, wie bei Newton-Raphson-basierten Verfahren, vollständig umgangen werden. Mit der derzeitigen Computertechnik können mit dem erfindungsgemäßen Verfahren deutlich kürzer und schneller auch Übergangszustände für Moleküle mit mehr als 100 Atomen berechnet werden, vor allem auch durch die Reduktion von personellem Aufwand der zur Bearbeitung entsprechender herkömmlicher Prozesse notwendig ist. Hierbei werden auch Computer Ressourcen gespart..

In **Schritt A** des erfindungsgemäßen Verfahrens wird zunächst eine Startgeometrie erzeugt, indem in Schritt A1 die dreidimensionale Darstellung mindestens eines Moleküls im energetischen Grundzustand bereitgestellt wird. Dann wird in Schritt A2 eine Bindung des Moleküls ausgewählt und eine Länge der Bindung, die von der Länge der Bindung im energetischen Grundzustand abweicht, so dass eine Startgeometrie erhalten wird. Bei den ausgewählten Bindungen handelt es sich bevorzugt um diejenigen Bindungen welche in der zu betrachtenden chemischen Reaktion gebildet oder gebrochen werden. Die Bindungslänge, die von der Länge der Bindung im energetischen Grundzustand abweicht ist um 10 bis 90% bevorzugt 20% bis 40 % größer als die Bindung im energetischen Grundzustand.

Bevorzugt weist das mindestens eine Molekül aus Schritt A1 eine Größe von mehr als 100 Atomen auf, weiter bevorzugt von mehr als 80 Atomen, bevorzugter von mehr als 60 Atomen und/oder beträgt die Länge der in Schritt **A2** ausgewählten Bindung höchstens 230 pm, bevorzugt höchstens 200 pm, weiter bevorzugt höchstens 180 pm, bevorzugter 150 pm.

Bevorzugt wird Schritt A durch einen Nutzer ausgeführt, der die dreidimensionale Darstellung eines Moleküls im energetischen Grundzustand in Schritt A1, sowie die Auswahl einer Bindung und der Länge der Bindung in Schritt A2 und die Darstellung der Startgeometrie in Schritt A3 in eine Eingabemaske für das computerimplementierte Verfahren eingibt.

In einer weiteren bevorzugten Ausführungsform des erfindungsgemäßen Verfahrens werden in Schritt **A1** mindestens zwei Moleküle I und II bereitgestellt und in Schritt **A2** können alternativ oder zusätzlich zu der mindestens einen Bindung auch mindestens ein Abstand zwischen mindestens einem Atom aus Molekül I und mindestens einem Atom aus Molekül II sowie die Länge des mindestens einen Abstands ausgewählt werden, wobei die Länge des Abstands insbesondere höchstens 230 pm beträgt, bevorzugt höchstens 200 pm, weiter bevorzugt höchstens 180 pm, bevorzugter 150 pm. Wenn das Verfahren mit mindestens zwei Molekülen I und II durchgeführt wird und die Länge des Abstands zwischen Atomen der verschiedenen Moleküle ausgewählt wird, so kann bevorzugt der Übergangszustand eine Synthesereaktion mit dem Verfahren berechnet werden. Bevorzugt weist Molekül I eine Größe von 2 bis 1000 Atomen auf und Molekül II eine Größe von 2 bis 1000 Atomen auf, bevorzugter weist Molekül I eine Größe von 2 bis 800 Atomen auf und Molekül II eine Größe von 2 bis 800 Atomen auf, noch bevorzugter weist Molekül I eine Größe von 2 bis 600 Atomen auf und Molekül II eine Größe von 2 bis 600 Atomen auf. Bevorzugt beträgt die Summe der Atome aus Molekül I und aus Molekül II mehr als 100 Atome.

Bevorzugt ist Molekül I ein Katalysator für die chemische Reaktion, insbesondere für eine Polymersynthese, und Molekül II ein Edukt der chemischen Reaktion. Dabei handelt es sich bei der Polymersynthese in dieser Ausführungsform bevorzugt um Polyurethansynthesen. Die chemische Reaktion in dieser Ausführungsform sind bevorzugt auch Synthesen von Monomeren für Polymerisationsreaktionen, industriell benötigte Grundchemikalien, Additiven, Tensiden und pharmakologischen Wirkstoffen. Insbesondere ist die chemische Reaktion eine Synthese für mit Katalysatoren erhaltene Grundchemikalien oder Edukte für chemische Synthesen. Unter Additiven werden allgemein Additive für Kunststoffe wie Weichmacher, Antioxidantien, Stellmittel verstanden, sowie Zusätze für Kraftstoffe, bei deren Synthese jeweils Katalysatoren verwendet werden.

In Schritt A3 wird die in Schritt A2 ausgewählte Startgeometrie in kartesischen und/oder internen Koordinaten dargestellt. Interne Koordinaten beschreiben die räumliche Anordnung der Atome relativ zueinander unter Benutzung von Bindungslängen, Bindungswinkel und Torsionswinkel.

In **Schritt B** des erfindungsgemäßen Verfahrens wird eine optimierte Startgeometrie ermittelt. Dazu wird zunächst in Schritt B1 ein Funktionsraum festgelegt. Der Funktionsraum umfasst die Raumkoordinaten von ausgewählten Atomen, wobei die Raumkoordinaten im Vektorraum aller im Molekül beinhalteten Atomkoordinaten einen Unterraum aufspannen. Die ausgewählten Atome für den Funktionsraum sind ein Satz an Atomen, die an einer Bindungsdissoziation beteiligt sind oder bevorzugt an einer Synthesereaktion. Des Weiteren umfasst der Funktionsraum die mindestens eine Bindung aus Schritt A2. Die Bei der bevorzugten Ausführungsform mit mindestens zwei Molekülen I und II umfasst der Funktionsraum den Abstand zwischen mindestens einem Atom aus Molekül I und mindestens einem Atom aus Molekül II.

Im folgenden Schritt B2 wird die Startgeometrie einer Geometrieoptimierung mittels einer quantenchemischen Methode unterzogen mit der Randbedingung, dass die in Schritt A2 ausgewählte Länge der mindestens einen Bindung konstant gehalten wird, so dass eine optimierte Startgeometrie erhalten wird. Dabei umfasst die Geometrieoptimierung alle Atome des als Startgeometrie ausgewählten Moleküls. Bei der bevorzugten Ausführungsform mit mindestens zwei Molekülen I und II wird bei der Geometrieoptimierung der Abstand zwischen mindestens einem Atom aus Molekül I und mindestens einem Atom aus Molekül II konstant gehalten, so dass eine optimierte Startgeometrie erhalten wird.

Bei der Geometrieoptimierung mit Randbedingung wird die Gesamtenergie des Moleküls als Funktion der Kernkoordinaten der im Molekül enthaltenen Atome mit einem quantenchemischen Verfahren minimiert. Um ein lokales Energieminimum zu erhalten wird die Energie nach Gradienten minimiert (zb. Steepest Descent), wobei die Energie soweit minimiert wird, wie die Randbedingung dies zulässt.

In Schritt B3 wird die Gradient-Norm B3 für die zuvor erhaltene optimierte Startgeometrie ermittelt, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Moleküls in der optimierten Startgeometrie. In Schritt B3 wird die gleiche quantenchemische Methode verwendet wie in Schritt B2. Die euklidische Norm dieses Vektors ist die Gradient-Norm. Mit anderen Worten wird der Gradient-Vektor der Energie berechnet. Der Gradient-Vektor spannt denselben vektoriellen Raum auf wie das Molekül. Jede Komponente des Vektors besteht aus der partiellen Ableitung der Energie in der $x_i$-Koordinaten ( $\frac{\partial}{\partial x_i} E(x)$ ) und spannt sich in die Richtung des Einheitsvektors der $x_i$-Koordinaten. Danach wird die (euklidische) Norm dieses Vektors ermittelt.

Die weitere Fortführung des Verfahrens von der Größe der erhaltenen Gradient-Norm B3 ab. Sofern die Gradient-Norm B3 $\nabla$ $0 \leq \nabla \leq 0{,}03$ $E_h$ $a_0^{-1}$ beträgt, dann kann die optimierten Startgeometrie als eine Vorstufe für den Übergangszustand der chemischen Reaktion eingeordnet werden und Schritt C des Verfahrens ausgelassen werden und das Verfahren mit Schritt D1 fortgeführt werden. Beträgt die Gradient-Norm B3 $\nabla > 0{,}03$ $E_h$ $a_0^{-1}$, dann wird Schritt C des Verfahrens durchgeführt und zunächst eine Vorstufe für den Übergangszustand der chemischen Reaktion ermittelt und zwar ausgehend von der optimierten Startgeometrie. Dabei steht $E_h$ für die Hartree Energie, wobei $1\ E_h = 4{,}3597 \cdot 10^{-18}$ J, und $a_0$ steht für den Bohr-Radius, wobei $1\ a_0 = 5{,}29 \cdot 10^{-11}$ m.

In **Schritt C** wird die Vorstufe für den Übergangszustand der chemischen Reaktion ermittelt. Dazu wird die optimierte Startgeometrie unter Anwendung eines Monte-Carlo-Algorithmus variiert indem zufällig generierte Auslenkungen von Atomen in einem Ensemble, das aus einem Molekül oder bevorzugt einer Geometrie von mindestens zwei Molekülen I und II besteht, vorgenommen.

Monte-Carlo-Algorithmen oder -Verfahren sind Simulationsverfahren, die analytisch nicht oder nur schwer lösbare mathematische Probleme durch nummerische Näherungen lösen. Dabei wird ein wahrscheinlichster Verlauf eines Experiments durch eine Vielzahl an zufällig angeordneten Einzelexperimenten beschrieben.

In dem erfindungsgemäßen in Schritt C angewandten Monte-Carlo-Algorithmus werden zufällig generierte Auslenkungen von Atomen in einem Ensemble, das aus einem Molekül oder einer Geometrie von mindestens zwei Molekülen I und II besteht, vorgenommen und die Änderung einer Observablen beobachtet. Bei dieser Observablen handelt es sich um eine Gradient-Norm. Dabei variiert das erfindungsgemäße Verfahren nur einen Unterraum der deutlich niedriger dimensional ist als der gesamte Funktionsraum des Ensembles und betrachtet die Änderung der Observablen des gesamten Ensembles als Funktion des reduzierten Funktionsraums.

Dazu werden aus dem in Schritt B1 ausgewählten Funktionsraum, in Schritt C 1.1 mindestens ein Atom ausgewählt. Dies geschieht mit Hilfe einer Zufallszahl Z1. Die Richtung einer Auslenkung des durch Z1 gewählten Atoms, bzw. die Richtung des Vektors für die Auslenkung, wird in Schritt C1.2 durch eine weitere Zufallszahl Z2 ausgewählt. Die Richtung wird bevorzugt definiert, indem im bevorzugten Schritt C1.2a die Position eines anderen Atoms als dem in Schritt C1.1. ausgewählten aus dem reduzierten Funktionsraum ausgewählt wird und dann im bevorzugten Schritt C1.2b die Richtung der Auslenkung ausgewählt wird, wobei die Position des in Schritt C1.2a ausgewählten Atoms die Richtung des Vektors bestimmt. Die Amplitude der Auslenkung wird bevorzugt durch eine dritte Zufallszahl Z3 im bevorzugten Schritt C1.2c bestimmt.

In einer bevorzugten Ausführungsform des erfindungsgemäßen Verfahrens umfasst Schritt C1.2 die weiteren folgenden Schritte:

C1.2a    Bestimmung eines weiteren Atoms aus dem in Schritt B1 ausgewählten Funktionsraum, das nicht mit dem in Schritt C1.1 ausgewählten Atom übereinstimmt,

C1.2b    Auswahl der Richtung des Vektors, wobei die Position des in Schritt C1.2.a ausgewählten Atoms die Richtung des Vektors bestimmt,

C1.2.c    zufällige Auswahl der Länge des Vektors, wobei der Wert für die Länge des Vektors positive oder negative Werte annehmen kann.

Die Gewichtung des zufällig ausgewählten Betrags des Vektors in Schritt C1.2 erfolgt bevorzugt durch Multiplikation des Zahlenwerts des zufällig ausgewählten Betrags des Vektors mit dem Zahlenwert der Gradient-Norm B3.

Dadurch, dass die Position des in Schritt C1.2 ausgewählten Atoms die Richtung des Vektors bestimmt, wird der Funktionsraum möglichst stark beschränkt und diese Vorgehensweise führt auch dazu das vorrangig Bindungslängen variiert werden, die Längen aufweisen wie sie sehr wahrscheinlich tatsächlich während einer Reaktion erreicht werden.

In Schritt C1.3 wird das in Schritt C.1.1 ausgewählte Atom anhand des Vektors aus Schritt C 1.2 aus seiner Position in der optimierten Startgeometrie ausgelenkt, so dass eine Vorstufe für den Übergangszustand der chemischen Reaktion erhalten wird.

Die erhaltene Vorstufe für den Übergangszustand wird in Schritt C2 einer Geometrieoptimierung mittels der quantenchemischen Methode unterzogen mit der Randbedingung, dass die mindestens eine Bindung aus Schritt A2 die Länge hat, die in Schritt C1.3 ermittelt wurde. In Schritt C2 wird die gleiche quantenchemische Methode verwendet wie in den Schritten B2 und B3. Dann wird in Schritt C3 die Gradient-Norm C3 für die Vorstufe für den Übergangszustand aus Schritt C2 ermittelt, wobei die Gradient-Norm durch die erste Ableitung der Funktion $E = f(x)$ mittels der quantenchemischen Methode erhalten wird, mit E=Gesamtenergie der Vorstufe für den Übergangszustand und x=Kernkoordinaten des Moleküls in der Vorstufe für den Übergangszustand. In Schritt C3 wird die gleiche quantenchemische Methode verwendet wie in den Schritten B2, B3 und C2.

Die weitere Fortführung des Verfahrens hängt von der Größe der erhaltenen Gradient-Norm C3 ab. Sofern die Gradient-Norm C3 $\nabla$ $0 \leq \nabla \leq 0{,}03$ $E_h\,a_0^{-1}$ beträgt, dann kann Schritt D1 des Verfahrens mit der Vorstufe für den Übergangszustand durchgeführt werden. Dabei steht $E_h$ für die Hartree Energie, wobei 1 $E_h$ = 4,3597 · 10$^{-18}$ J, und $a_0$ steht für den Bohr-Radius, wobei 1 $a_0$ = 5,29 · 10$^{-11}$ m.

Beträgt die Gradient-Norm C3 $\nabla > 0{,}03$ $E_h\,a_0^{-1}$, dann werden in Schritt 4.2 die Schritte C1 bis C3 wiederholt bis eine Gradient-Norm C3 $\nabla$ $0 \leq \nabla \leq 0{,}03$ $E_h\,a_0^{-1}$ erhalten wird. Durch die Wiederholung der Schritte C1 bis C3 wird ein iteratives Verfahren dargestellt, mit dem die Gradient-Norm minimiert werden soll. Daher soll als Ausgangspunkt für die Wiederholung des Verfahrens jeweils eine Molekülgeometrie gewählt werden, die bereits eine möglichst niedrige Gradient-Norm aufweist. Bei der Wiederholung der Schritte C1 bis C3 kann also in Schritt C1 nicht nur die optimierte Startgeometrie variiert werden, sondern auch eine Vorstufe für den Übergangszustand, sofern der Wert ihrer Gradient-Norm C3 kleiner ist als derjenige der optimierten Startgeometrie. Bevorzugt werden die bei der Durchführung des Verfahrens erhaltene optimierte Startgeometrie und erhaltene(n) Vorstufe(n) für den Übergangszustand sowie die Werte der jeweiligen Gradient-Normen C3 gespeichert.

In dem Fall, dass die Schritte C1 bis C3 ein Mal durchgeführt wurden, kann aus zwei Molekülgeometrien zur Durchführung der Wiederholung diejenige mit der niedrigsten Gradient-Norm ausgewählt werden, nämlich entweder die optimierte Startgeometrie oder die in ersten Durchgang erhaltene Vorstufe für den Übergangszustand. In dem Fall, dass die Schritte C1 bis C3 ein Mal durchgeführt wurden, wird in Schritt C1 die Geometrie der Vorstufe für den Übergangszustand variiert, wenn der Wert ihrer Gradient-Norm C3 niedriger ist als der Wert der Gradient-Norm B3 der optimierten Startgeometrie.

In dem Fall, dass die Schritte C1 bis C3 schon durchgeführt wurden, kann aus mehr als zwei Molekülgeometrien zur Durchführung der Wiederholung diejenige mit der niedrigsten Gradient-Norm ausgewählt werden, nämlich entweder die optimierte Startgeometrie oder die in bei den Wiederholungen erhaltenen (mehr als eine) Vorstufen für den Übergangszustand. In dem Fall dass die Schritte C1 bis C3 mehr als ein Mal durchgeführt wurden, wird in Schritt C1 die Geometrie der optimierten Startgeometrie oder derjenigen Vorstufe für den Übergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist.

Bevorzugt ist in Schritt **C4.1** die Gradient-Norm C3 $\leq 0{,}02$ $E_h\,a_0^{-1}$, bevorzugter C3 $\leq 0{,}01$ $E_h\,a_0^{-1}$, und Schritt **C4.2** wird so lange durchgeführt, bis eine Gradient-Norm C3 $\leq 0{,}02$ $E_h\,a_0^{-1}$, bevorzugter C3 $\leq 0{,}01$ $E_h\,a_0^{-1}$, erhalten wird.

Bevorzugt wird Schritt **C4.2** höchstens 50 Mal, bevorzugt höchstens 40 Mal, bevorzugter höchstens 30 Mal, noch bevorzugter höchstens 10 Mal, insbesondere höchstens 3 Mal, wiederholt. Bevorzugt kann bei der Durchführung von Schritt **C4.2** bei der Wiederholung von Schritt **C1** ein anderes Atom ausgewählt werden als bei der vorrangegangenen Durchführung des Verfahrens.

Ein Vorteil des erfindungsgemäßen Verfahrens ist, dass das bei der Variation unter Anwendung eines Monte-Carlo-Algorithmus nur die Atomkoordinaten des ausgewählten Funktionsraums, also des Unterraums der durch die ausgewählten, an der betrachteten Bindungsdissoziation oder bevorzugt Synthesereaktion beteiligten Atome im Vektorraum aller im Molekül, oder im Molekülensemble, beinhalteten Atomkoordinaten aufgespannt wird. Das heißt, der erfindungsgemäße Monte-Carlo-Algorithmus operiert nur in diesem Unterraum, da seine Funktionen nur hierin definiert sind. Damit ist der Unterraum der an der Dissoziation, oder bevorzugten Synthesereaktion, beteiligten Koordinaten der Funktionsraum des Monte-Carlo-Algorithmus. Die Berechnung der Energien und der Gradient-Vektoren (bzw. deren Norm) hängen zwar von den gesamten Koordinaten im Molekül ab, jedoch betrachtet der erfindungsgemäße Monte-Carlo-Algorithmus diese nur in Abhängigkeit der Koordinaten des Unterraumes. Damit bei dieser Betrachtung der Funktionswert, die zu minimierende Gradient-Norm, eindeutig bleibt, wird vor jeder Betrachtung des Funktionswerts die gesamte Molekülstruktur durch eine Geometrieoptimierung mit Randbedingung(en) relaxiert. Die Randbedingung(en) sind dabei die durch den Monte-Carlo-Algorithmus erzeugten Bindungsabstände.

Ist die Gradient- Norm ausreichend minimiert, wird die Eigenschaft der negativen Krümmung im Bereich des erhaltenen stationären Punkts durch infinitesimale Auslenkungen und anschließender Geometrieoptimierung in **Schritt D** verifiziert. Dabei muss die Auslenkung entlang der Bindungsdissoziation eine Abnahme der Gesamtenergie ergeben. Ein weiterer Vorteil der vorliegenden Erfindung ist, dass auf diese Weise die aufwendige Berechnung der zweiten Ableitungen der Energie nach den Kernkoordinaten vollständig umgangen werden kann.

Bevorzugt wird in Schritt **D3** die Energie der zwei optimierten Vorstufen (i) und (II) in der Einheit der Einheit $E_h\, a_0^{-1}$ dargestellt.

Eine Vorstufe wird in Schritt **D4.1** als Übergangszustand eingeordnet, wenn sowohl der Wert der Energie der optimierten Vorstufe (i) als auch der Wert der Energie der optimierten Vorstufe (ii) beide kleiner sind als der Wert der Energie C3 oder B3 der Vorstufe, die in Schritt D1 verwendet wurde. Wenn einer der beiden Werte der Energie der optimierten Vorstufe (i) oder (ii) größer ist, oder beide Werte der Energie der optimierten Vorstufen (i) und (ii) größer sind, als der Wert der Energie C3 oder B3 der Vorstufe, die in Schritt D1 verwendet wurde, so wird das Verfahren ab Schritt C wiederholt. Bevorzugt wird das Verfahren ab Schritt C höchstens 10 Mal, bevorzugt höchstens 3 Mal wiederholt.

In den Schritten **B2, B3, C2, C3, D2 und D3** wird jeweils dieselbe quantenchemische Methode verwendet. Bevorzugt ist die quantenchemische Methode aus Schritten **B2, B3, C2, C3, D2** und **D3** eine semiempirische Methode, Dichte-Funktional-theoretische Methode oder eine Annäherung der Schrödinger-Gleichung, insbesondere sind Dichte-Funktional-theoretische Methoden bevorzugt, wie beispielsweise das TPSS-Dichtefunktional mit einem def2-SVP-Basissatz, so wie standardmäßig im Turbomole-Programmpaket implementiert. In einer bevorzugten Ausführungsform werden die quantenmechanischen Berechnungen mit dem Programmpaket TURBOMOLE durchgeführt. Bevorzugt wird zur Berechnung ein Computer mit 16 Core-Prozessoren mit einer Taktfrequenz von 3.20GHz und 25MB Zwischenspeicher mit einem RAM Speicher von 128 GB DDR4 2400 rg ECC verwendet.

Bevorzugt ist die chemische Reaktion eine Synthese ausgewählt aus der Gruppe bestehend aus Polymersynthesen, insbesondere Polyurethansynthesen, Synthesen von Monomeren für Polymerisationsreaktionen, industriell benötigte Grundchemikalien, Additiven, Tensiden und pharmakologischen Wirkstoffen. Insbesondere ist die chemische Reaktion eine Synthese für mit Katalysatoren erhaltene Grundchemikalien oder Edukte für chemische Synthesen. Unter Additiven werden allgemein Additive für Kunststoffe wie Weichmacher, Antioxidantien, Stellmittel verstanden, sowie Zusätze für Kraftstoffe, bei deren Synthese jeweils Katalysatoren verwendet werden.

Wenn bei der bevorzugten Ausführungsform des Verfahrens mindestens zwei Moleküle I und **II** bereitgestellt werden, können bevorzugt die unter den Buchstaben **A, B,** und **C** des Verfahrens genannten Schritte wiederholt werden, wobei bei jeder Wiederholung im Vergleich zur vorrangegangenen Durchführungen des Verfahrens

Molekül I verändert oder ein anderes Molekül als Molekül I bereitgestellt wird, und
Molekül II nicht verändert und auch kein anderes Molekül als Molekül II bereitgestellt wird, und der zusätzliche Schritt D0 durchgeführt wird:
**D0** Vergleich der bei den Wiederholungen erhaltenen Gradienten-Norm C3 für die verschiedenen Vorstufen für den Übergangszustand und Auswahl der Vorstufe für den Übergangszustand mit der niedrigsten Gradienten-Norm C3 und Durchführung von Schritt D1 und/oder D2 mit der ausgewählten Vorstufe für den Übergangszustand.

Bei dieser zuvor beschriebenen bevorzugten Ausführungsform des Verfahrens werden zunächst für unterschiedliche Kombinationen von Molekülen die Gradient-Norm C3 berechnet und die Ergebnisse gespeichert. Dann können in einem bevorzugten Schritt D0 die erhaltenen Werte für die Gradient-Norm C3 verglichen werden und die Kombination von Molekülen mit der niedrigsten Gradienten-Norm C3 ausgewählt werden. Diese bevorzugte Ausführungsform des Verfahrens ermöglicht also den direkten Vergleich von unterschiedlichen Kombinationen von Molekülen.

Ein weiterer Gegenstand der Erfindung ist ein System zur Datenverarbeitung umfassend Mittel zur Ausführung eines erfindungsgemäßen Verfahrens.

Darüber hinaus sind weitere Gegenstände der Erfindung ein Computerprogramm und ein computerlesbares Speichermedium, umfassend Befehle, die bei der Ausführung des Programms durch einen Computer diesen veranlassen, die folgenden Schritte eines Verfahrens durchzuführen:

**A Erzeugen einer Startgeometrie**

A1 Bereitstellung der dreidimensionalen Darstellung mindestens eines Moleküls im energetischen Grundzustand,

A2 Auswahl von mindestens einer Bindung des mindestens einen Moleküls und Auswahl einer Länge der Bindung, wobei die ausgewählte Länge nicht der Länge der Bindung im energetischen Grundzustand des Moleküls entspricht, so dass eine Startgeometrie für die chemische Reaktion erhalten wird,

A3 Dreidimensionale Darstellung der Startgeometrie in kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst,

B2 Geometrieoptimierung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) der quantenchemischen Methode und erhalten wird, mit E=Gesamtenergie der optimierten Startgeometrie und x=Kernkoordinaten des Moleküls in der optimierten Startgeometrie,

B4.1 wenn die Gradient-Norm B3 $\nabla$ $0 \leq \nabla \leq 0{,}03$ $E_h$ $a_0^{-1}$ beträgt, dann Einordnung der optimierten Startgeometrie als eine Vorstufe für den Übergangszustand der chemischen Reaktion und Fortführung des Verfahrens mit Schritt D1, oder

B4.2 wenn die Gradient-Norm B3 $\nabla > 0{,}03$ $E_h$ $a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

**C Ermittlung der Vorstufe für den Übergangszustand der chemischen Reaktion**

C1 Variation der optimierten Startgeometrie unter Anwendung eines Monte-Carlo-Algorithmus wobei

C1.1 wenigstens ein Atom aus dem in Schritt B1 ausgewählten Funktionsraum zufällig ausgewählt wird,

C1.2 ein Vektor für eine Auslenkung des in Schritt C1 gewählten Atoms zufällig ausgewählt wird, wobei der zufällig ausgewählte Betrag des Vektors mit der Gradient-Norm B3 gewichtet wird,

C1.3 das in Schritt C1.1 ausgewählte Atom anhand des Vektors aus Schritt C1.2 aus der Position des Atoms in der optimierten Startgeometrie ausgelenkt wird, so dass eine Vorstufe für einen Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schritt A2 die Länge hat, die in Schritt C3 ermittelt wurde,

C3 Ermittlung der Gradient-Norm C3 für die Vorstufe für den Übergangszustand aus Schritt C2, wobei die Gradient-Norm durch die erste Ableitung der Funktion E = f(x) der quantenchemischen Methode und erhalten wird, mit E=Gesamtenergie der Vorstufe für den Übergangszustand und x=Kernkoordinaten des Moleküls in der Vorstufe für den Übergangszustand,

C4.1 wenn die Gradient-Norm C3 $\nabla$ $0 \leq \nabla \leq 0{,}03$ $E_h$ $a_0^{-1}$ beträgt, dann Fortführung des Verfahrens mit Schritt D1, oder

C4.2 wenn die Gradient-Norm C3 $\nabla > 0{,}03$ $E_h$ $a_0^{-1}$ $E_h$ $a_0^{-1}$ beträgt, dann Wiederholung der Schritte C1 bis C3 bis eine Gradient-Norm C3 $\nabla$ $0 \leq \nabla \leq 0{,}03$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Variation der Vorstufe aus Schritt C4.1 oder der Vorstufe aus Schritt B4.1, indem folgende Schritte für ein Atom innerhalb des in B1 definierten Funktionsraums durchgeführt werden

D1.1 Auswahl eines Atoms aus dem in Schritt B1 ausgewählten Funktionsraum,

D1.2 Auswahl eines Vektors für eine Auslenkung des in Schritt D1.1 gewählten Atoms

D1.3 Auslenkung des in Schritt D1.1 ausgewählten Atoms anhand des Vektors aus Schritt D1.2 aus seiner Position in der Vorstufe, wobei die Position einmal um einen positiven Wert des Betrags des Vektors und einmal um einen negativen Wert des Betrags des Vektors ausgelenkt wird, so dass bei der Durchführung der Schritte D1.1 bis D.1.3 zwei ausgelenkte Vorstufen erhalten werden,

D2 Geometrieoptimierung der zwei ausgelenkten Vorstufen aus Schritt D1 mittels der quantenchemischen Methode unter der Randbedingung dass die in D1 erhaltenen Bindungsabstände konstant gehalten werden, so dass zwei optimierte Vorstufen (i) und (ii) erhalten werden,

D3 Berechnung der Energie der zwei optimierten Vorstufen (i) und (ii) aus Schritt D2 mittels des quantenchemischen Verfahrens,

D4 Vergleich des Wertes der Energie der zwei optimierten Vorstufen mit dem Wert der Energie C3 oder B3 der Vorstufe, die in Schritt D1 verwendet wurde,

D4.1. wenn der Wert der Energie der optimierten Vorstufe (i) und der Wert der Energie der optimierten Vorstufe (ii) jeweils kleiner sind als der Wert der Energie C3 oder B3 der Vorstufe, die in Schritt D1 verwendet wurde, dann Einordnung der Vorstufe, die in Schritt D1 verwendet wurde, als Übergangszustand,

D4.2. wenn der Wert der Energie der optimierten Vorstufe (i) oder der Wert der Energie der optimierten Vorstufe (ii) nicht jeweils kleiner ist als der Wert der EnergieC3 oder B3 der Vorstufe, die in Schritt D1 verwendet wurde, dann Wiederholung des Verfahrens ab Schritt C1.

Bevorzugt umfasst das Computerprogramm und/oder das computerlesbare Speichermedium Befehle, die bei der Ausführung des Programms durch einen Computer diesen veranlassen, die Schritte B bis D des Verfahrens durchzuführen.

Des Weiteren ist die Erfindung auf die Verwendung des erfindungsgemäßen Computerprogramms oder des erfindungsgemäßen computerlesbaren Speichermediums zur Bewertung von Übergangszuständen einer chemischen Reaktion, insbesondere einer Polymersynthese gerichtet.

Die Erfindung betrifft insbesondere die folgenden Ausführungsformen:

In einer ersten Ausführungsform betrifft die Erfindung ein computerimplementiertes Verfahren zur Berechnung von Übergangszuständen einer chemischen Reaktion umfassend die Schritte

## A Erzeugen einer Startgeometrie

A1 Bereitstellung der dreidimensionalen Darstellung mindestens eines Moleküls im energetischen Grundzustand,

A2 Auswahl von mindestens einer Bindung des mindestens einen Moleküls und Auswahl einer Länge der Bindung, wobei die ausgewählte Länge nicht der Länge der Bindung im energetischen Grundzustand des Moleküls entspricht, so dass eine Startgeometrie für die chemische Reaktion erhalten wird,

A3 Dreidimensionale Darstellung der Startgeometrie in kartesischen und/oder internen Koordinaten,

## B Ermittlung einer optimierten Startgeometrie

B1 Festlegung eines Funktionsraumes welcher die mindestens eine Bindung aus Schritt A2 und die durch diese Bindung verbundenen Atome umfasst,

B2 Geometrieoptimierung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) der quantenchemischen Methode und erhalten wird, mit E=Gesamtenergie der optimierten Startgeometrie und x=Kernkoordinaten des Moleküls in der optimierten Startgeometrie,

B4.1 wenn die Gradient-Norm B3 $\nabla$ $0 \leq \nabla \leq 0{,}03$ $E_h\, a_0^{-1}$ beträgt, dann Einordnung der optimierten Startgeometrie als eine Vorstufe für den Übergangszustand der chemischen Reaktion und Fortführung des Verfahrens mit Schritt D1, oder

B4.2 wenn die Gradient-Norm B3 $\nabla > 0{,}03$ $E_h\, a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

## C Ermittlung der Vorstufe für den Übergangszustand der chemischen Reaktion

C1 Variation der optimierten Startgeometrie unter Anwendung eines Monte-Carlo-Algorithmus wobei

C1.1 wenigstens ein Atom aus dem in Schritt B1 ausgewählten Funktionsraum zufällig ausgewählt wird,

C1.2 ein Vektor für eine Auslenkung des in Schritt C1 gewählten Atoms zufällig ausgewählt wird, wobei der zufällig ausgewählte Betrag des Vektors mit der Gradient-Norm B3 gewichtet wird,

C1.3 das in Schritt C1.1 ausgewählte Atom anhand des Vektors aus Schritt C1.2 aus der Position des Atoms in der optimierten Startgeometrie ausgelenkt wird, so dass eine Vorstufe für einen Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schritt A2 die Länge hat, die in Schritt C3 ermittelt wurde,

C3 Ermittlung der Gradient-Norm C3 für die Vorstufe für den Übergangszustand aus Schritt C2, wobei die Gradient-Norm durch die erste Ableitung der Funktion E = f(x) der quantenchemischen Methode und erhalten wird, mit E=Gesamtenergie der Vorstufe für den Übergangszustand und x=Kernkoordinaten des Moleküls in der Vorstufe für den Übergangszustand,

C4.1 wenn die Gradient-Norm C3 $\nabla$ $0 \leq \nabla \leq 0{,}03$ $E_h$ $a_0^{-1}$ beträgt, dann Fortführung des Verfahrens mit Schritt D1, oder

C4.2 wenn die Gradient-Norm C3 $\nabla > 0{,}03$ $E_h$ $a_0^{-1}$ $E_h$ $a_0^{-1}$ beträgt, dann Wiederholung der Schritte C1 bis C3 bis eine Gradient-Norm C3 $\nabla$ $0 \leq \nabla \leq 0{,}03$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

## D Ermittlung des Übergangszustandes

D1 Variation der Vorstufe aus Schritt C4.1 oder der Vorstufe aus Schritt B4.1, indem folgende Schritte für ein Atom innerhalb des in B1 definierten Funktionsraums durchgeführt werden

D1.1 Auswahl eines Atoms aus dem in Schritt B1 ausgewählten Funktionsraum,

D1.2 Auswahl eines Vektors für eine Auslenkung des in Schritt D1.1 gewählten Atoms,

D1.3 Auslenkung des in Schritt D1.1 ausgewählten Atoms anhand des Vektors aus Schritt D1.2 aus seiner Position in der Vorstufe, wobei die Position einmal um einen positiven Wert des Betrags des Vektors und einmal um einen negativen Wert des Betrags des Vektors ausgelenkt wird, so dass bei der Durchführung der Schritte D1.1 bis D.1.3 zwei ausgelenkte Vorstufen erhalten werden,

D2 Geometrieoptimierung der zwei ausgelenkten Vorstufen aus Schritt D1 mittels der quantenchemischen Methode unter der Randbedingung dass die in D1 erhaltenen Bindungsabstände konstant gehalten werden, so dass zwei optimierte Vorstufen (i) und (ii) erhalten werden,

D3 Berechnung der Energie der zwei optimierten Vorstufen (i) und (ii) aus Schritt D2 mittels des quantenchemischen Verfahrens,

D4 Vergleich des Wertes der Energie der zwei optimierten Vorstufen mit dem Wert der Energie C3 oder B3 der Vorstufe, die in Schritt D1 verwendet wurde,

D4.1. wenn der Wert der Energie der optimierten Vorstufe (i) und der Wert der Energie der optimierten Vorstufe (ii) jeweils kleiner sind als der Wert der Energie C3 oder B3 der Vorstufe, die in Schritt D1 verwendet wurde, dann Einordnung der Vorstufe, die in Schritt D1 verwendet wurde, als Übergangszustand,

D4.2. wenn der Wert der Energie der optimierten Vorstufe (i) oder der Wert der Energie der optimierten Vorstufe (ii) nicht jeweils kleiner ist als der Wert der Energie C3 oder B3 der Vorstufe, die in Schritt D1 verwendet wurde, dann Wiederholung des Verfahrens ab Schritt C1.

In einer zweiten Ausführungsform betrifft die Erfindung ein Verfahren nach Ausführungsform 1, dadurch gekennzeichnet, dass das mindestens eine Molekül aus Schritt A1 eine Größe von mehr als 100 Atomen aufweist und/oder dass die Länge der in Schritt **A2** ausgewählten Bindung höchstens 230 pm beträgt.

In einer dritten Ausführungsform betrifft die Erfindung ein Verfahren nach einer der Ausführungsformen 1 oder 2, dadurch gekennzeichnet, dass Schritt **C1.2** die weiteren folgenden Schritte umfasst:

C1.2a    Bestimmung eines weiteren Atoms aus dem in Schritt B1 ausgewählten Funktionsraum, das nicht mit dem in Schritt C1.1 ausgewählten Atom übereinstimmt,

C1.2b    Auswahl der Richtung des Vektors, wobei die Position des in Schritt C1.2.a ausgewählten Atoms die Richtung des Vektors bestimmt,

C1.2.c    zufällige Auswahl der Länge des Vektors, wobei der Wert für die Länge des Vektors positive oder negative Werte annehmen kann.

In einer vierten Ausführungsform betrifft die Erfindung ein Verfahren nach einer der vorstehenden Ausführungsformen, dadurch gekennzeichnet, dass die Gewichtung des zufällig ausgewählten Betrags des Vektors in Schritt **C1.2** durch Multiplikation des Zahlenwerts des zufällig ausgewählten Betrags des Vektors mit dem Zahlenwert der Gradient-Norm B3 erfolgt.

In einer fünften Ausführungsform betrifft die Erfindung ein Verfahren nach einer der vorstehenden Ausführungsformen, dadurch gekennzeichnet, dass in Schritt **C4.1** die Gradient-Norm $C3 \leq 0{,}02\,E_h\,a_0^{-1}$, bevorzugt $\leq 0{,}01\,E_h\,a_0^{-1}$, ist und Schritt **C4.2** so lange durchgeführt wird, bis eine Gradient-Norm $C3 \leq 0{,}02\,E_h\,a_0^{-1}$, bevorzugt $\leq 0{,}01\,E_h\,a_0^{-1}$, erhalten wird.

In einer sechsten Ausführungsform betrifft die Erfindung ein Verfahren nach einer der vorstehenden Ausführungsformen, dadurch gekennzeichnet, dass Schritt **C4.2** höchstens 50 Mal, bevorzugt höchstens 30 Mal, insbesondere höchstens 3 Mal, wiederholt wird.

In einer siebten Ausführungsform betrifft die Erfindung ein Verfahren nach einer der vorstehenden Ausführungsformen, dadurch gekennzeichnet, dass bei der Durchführung von Schritt **C4.2** bei der Wiederholung von Schritt **C1** ein anderes Atom ausgewählt werden kann als bei der vorrangegangenen Durchführung des Verfahrens.

In einer achten Ausführungsform betrifft die Erfindung ein Verfahren nach einer der vorstehenden Ausführungsformen, dadurch gekennzeichnet, dass die quantenchemische Methode aus Schritten **B2, B3, C2, C3, D2** und **D3** eine semiempirische Methode, Dichte-Funktional-theoretische Methode oder eine Annäherung der Schrödinger-Gleichung ist, insbesondere ist die quantenchemische Methode aus Schritten **B2, B3, C2, C3, D2** und **D3** eine Dichte-Funktional-theoretische Methode.

In einer neunten Ausführungsform betrifft die Erfindung ein Verfahren nach einer der vorstehenden Ausführungsformen, dadurch gekennzeichnet, dass die chemische Reaktion eine Synthese ist ausgewählt aus der Gruppe bestehend aus Polymersynthesen, insbesondere Polyurethansynthesen, Synthesen von Monomeren für Polymerisationsreaktionen, industriell benötigte Grundchemikalien Plattformchemikalien, Additiven, Tensiden und pharmakologischen Wirkstoffen.

In einer zehnten Ausführungsform betrifft die Erfindung ein Verfahren nach einer der vorstehenden Ausführungsformen, dadurch gekennzeichnet, dass in Schritt **A1** mindestens zwei Moleküle I und II bereitgestellt werden und in Schritt **A2** alternativ oder zusätzlich zu der mindestens einen Bindung auch mindestens ein Abstand zwischen mindestens einem Atom aus Molekül I und mindestens einem Atom aus Molekül II sowie die Länge des mindestens einen Abstands ausgewählt werden, wobei die Länge des Abstands insbesondere höchstens 230 pm beträgt .

In einer elften Ausführungsform betrifft die Erfindung ein Verfahren nach Ausführungsform 10, dadurch gekennzeichnet, dass die unter den Buchstaben **A, B** und **C** des Verfahrens genannten Schritte wiederholt werden, wobei bei jeder Wiederholung im Vergleich zur vorrangegangenen Durchführungen des Verfahrens

Molekül I verändert wird oder ein anderes Molekül als Molekül I bereitgestellt wird, und

Molekül II nicht verändert wird und auch kein anderes Molekül als Molekül II bereitgestellt wird, und umfassend den zusätzlichen Schritt

**D0** Vergleich der bei den Wiederholungen erhaltenen Gradienten-Norm C3 für die verschiedenen Vorstufen für den Übergangszustand und Auswahl der Vorstufe für den Übergangszustand mit der niedrigsten Gradienten-Norm C3 und Durchführung von Schritt D1 und/oder D2 mit der ausgewählten Vorstufe für den Übergangszustand.

In einer zwölften Ausführungsform betrifft die Erfindung ein Verfahren nach einer der Ausführungsformen 10 oder 11, dadurch gekennzeichnet, dass Molekül I ein Katalysator für die chemische Reaktion ist, insbesondere für eine Polymersynthese, und Molekül II ein Edukt der chemischen Reaktion.

In einer dreizehnten Ausführungsform betrifft die Erfindung ein Verfahren nach einer der Ausführungsformen 10 bis 13, dadurch gekennzeichnet, dass Molekül I eine Größe von 2 bis 1000 Atomen aufweist und Molekül II eine Größe von 2 bis 1000 Atomen aufweist, bevorzugt soll die Summe der Atome aus Molekül I und aus Molekül II mehr als 100 Atome betragen.

In einer vierzehnten Ausführungsform betrifft die Erfindung System zur Datenverarbeitung umfassend Mittel zur Ausführung eines Verfahrens umfassend die Schritte

**A Erzeugen einer Startgeometrie**

A1 Bereitstellung der dreidimensionalen Darstellung mindestens eines Moleküls im energetischen Grundzustand,

A2 Auswahl von mindestens einer Bindung des mindestens einen Moleküls und Auswahl einer Länge der Bindung, wobei die ausgewählte Länge nicht der Länge der Bindung im energetischen Grundzustand des Moleküls entspricht, so dass eine Startgeometrie für die chemische Reaktion erhalten wird,

A3 Dreidimensionale Darstellung der Startgeometrie in kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst,

B2 Geometrieoptimierung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) der quantenchemischen Methode und erhalten wird, mit E=Gesamtenergie der optimierten Startgeometrie und x=Kernkoordinaten des Moleküls in der optimierten Startgeometrie,

B4.1 wenn die Gradient-Norm B3 $\nabla$ $0 \leq \nabla \leq 0{,}03$ $E_h$ $a_0^{-1}$ beträgt, dann Einordnung der optimierten Startgeometrie als eine Vorstufe für den Übergangszustand der chemischen Reaktion und Fortführung des Verfahrens mit Schritt D1, oder

B4.2 wenn die Gradient-Norm B3 $\nabla$ $> 0{,}03$ $E_h$ $a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

**C Ermittlung der Vorstufe für den Übergangszustand der chemischen Reaktion**

C1 Variation der optimierten Startgeometrie unter Anwendung eines Monte-Carlo-Algorithmus wobei

C1.1 wenigstens ein Atom aus dem in Schritt B1 ausgewählten Funktionsraum zufällig ausgewählt wird,

C1.2 ein Vektor für eine Auslenkung des in Schritt C1 gewählten Atoms zufällig ausgewählt wird, wobei der zufällig ausgewählte Betrag des Vektors mit der Gradient-Norm B3 gewichtet wird,

C1.3 das in Schritt C1.1 ausgewählte Atom anhand des Vektors aus Schritt C1.2 aus der Position des Atoms in der optimierten Startgeometrie ausgelenkt wird, so dass eine Vorstufe für einen Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schritt A2 die Länge hat, die in Schritt C3 ermittelt wurde,

C3 Ermittlung der Gradient-Norm C3 für die Vorstufe für den Übergangszustand aus Schritt C2, wobei die Gradient-Norm durch die erste Ableitung der Funktion E = f(x) der quantenchemischen Methode und erhalten wird, mit E=Gesamtenergie der Vorstufe für den Übergangszustand und x=Kernkoordinaten des Moleküls in der Vorstufe für den Übergangszustand,

C4.1 wenn die Gradient-Norm C3 $\nabla$ $0 \leq \nabla \leq 0{,}03$ $E_h$ $a_0^{-1}$ beträgt, dann Fortführung des Verfahrens mit Schritt D1, oder

C4.2 wenn die Gradient-Norm C3 $\nabla$ $> 0{,}03$ $E_h$ $a_0^{-1}$ $E_h$ $a_0^{-1}$ beträgt, dann Wiederholung der Schritte C1 bis C3 bis eine Gradient-Norm C3 $\nabla$ $0 \leq \nabla \leq 0{,}03$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Variation der Vorstufe aus Schritt C4.1 oder der Vorstufe aus Schritt B4.1, indem folgende Schritte für ein Atom innerhalb des in B1 definierten Funktionsraums durchgeführt werden

D1.1 Auswahl eines Atoms aus dem in Schritt B1 ausgewählten Funktionsraum,

D1.2 Auswahl eines Vektors für eine Auslenkung des in Schritt D1.1 gewählten Atoms

D1.3 Auslenkung des in Schritt D1.1 ausgewählten Atoms anhand des Vektors aus Schritt D1.2 aus seiner Position in der Vorstufe, wobei die Position einmal um einen positiven Wert des Betrags des Vektors und einmal um einen negativen Wert des Betrags des Vektors ausgelenkt wird, so dass bei der Durchführung der Schritte D1.1 bis D.1.3 zwei ausgelenkte Vorstufen erhalten werden,

D2 Geometrieoptimierung der zwei ausgelenkten Vorstufen aus Schritt D1 mittels der quantenchemischen Methode unter der Randbedingung dass die in D1 erhaltenen Bindungsabstände konstant gehalten werden, so dass zwei optimierte Vorstufen (i) und (ii) erhalten werden,

D3 Berechnung der Energie der zwei optimierten Vorstufen (i) und (ii) aus Schritt D2 mittels des quantenchemischen Verfahrens,

D4 Vergleich des Wertes der Energie der zwei optimierten Vorstufen mit dem Wert der Energie C3 oder B3 der Vorstufe, die in Schritt D1 verwendet wurde,

D4.1. wenn der Wert der Energie der optimierten Vorstufe (i) und der Wert der Energie der optimierten Vorstufe (ii) jeweils kleiner sind als der Wert der Energie C3 oder B3 der Vorstufe, die in Schritt D1 verwendet wurde, dann Einordnung der Vorstufe, die in Schritt D1 verwendet wurde, als Übergangszustand,

D4.2. wenn der Wert der Energie der optimierten Vorstufe (i) oder der Wert der Energie der optimierten Vorstufe (ii) nicht jeweils kleiner ist als der Wert der Energie C3 oder B3 der Vorstufe, die in Schritt D1 verwendet wurde, dann Wiederholung des Verfahrens ab Schritt C1.

In einer fünfzehnten Ausführungsform betrifft die Erfindung ein Computerprogramm, umfassend Befehle, die bei der Ausführung des Programms durch einen Computer diesen veranlassen, die folgenden Schritte eines Verfahrens durchzuführen

## A Erzeugen einer Startgeometrie

A1 Bereitstellung der dreidimensionalen Darstellung mindestens eines Moleküls im energetischen Grundzustand,

A2 Auswahl von mindestens einer Bindung des mindestens einen Moleküls und Auswahl einer Länge der Bindung, wobei die ausgewählte Länge nicht der Länge der Bindung im energetischen Grundzustand des Moleküls entspricht, so dass eine Startgeometrie für die chemische Reaktion erhalten wird,

A3 Dreidimensionale Darstellung der Startgeometrie in kartesischen und/oder internen Koordinaten,

## B Ermittlung einer optimierten Startgeometrie

B1 Festlegung eines Funktionsraumes welcher die mindestens eine Bindung aus Schritt A2 und die durch diese Bindung verbundenen Atome umfasst,

B2 Geometrieoptimierung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion $E = f(x)$ der quantenchemischen Methode und erhalten wird, mit E=Gesamtenergie der optimierten Startgeometrie und x=Kernkoordinaten des Moleküls in der optimierten Startgeometrie,

B4.1 wenn die Gradient-Norm B3 $\nabla$ $0 \leq \nabla \leq 0{,}03$ $E_h$ $a_0^{-1}$ beträgt, dann Einordnung der optimierten Startgeometrie als eine Vorstufe für den Übergangszustand der chemischen Reaktion und Fortführung des Verfahrens mit Schritt D1, oder

B4.2 wenn die Gradient-Norm B3 $\nabla > 0{,}03$ $E_h$ $a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

## C Ermittlung der Vorstufe für den Übergangszustand der chemischen Reaktion

C1 Variation der optimierten Startgeometrie unter Anwendung eines Monte-Carlo-Algorithmus wobei

C1.1 wenigstens ein Atom aus dem in Schritt B1 ausgewählten Funktionsraum zufällig ausgewählt wird,

C1.2 ein Vektor für eine Auslenkung des in Schritt C1 gewählten Atoms zufällig ausgewählt wird, wobei der zufällig ausgewählte Betrag des Vektors mit der Gradient-Norm B3 gewichtet wird,

C1.3 das in Schritt C1.1 ausgewählte Atom anhand des Vektors aus Schritt C1.2 aus der Position des Atoms in der optimierten Startgeometrie ausgelenkt wird, so dass eine Vorstufe für einen Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schritt A2 die Länge hat, die in Schritt C3 ermittelt wurde,

C3 Ermittlung der Gradient-Norm C3 für die Vorstufe für den Übergangszustand aus Schritt C2, wobei die Gradient-Norm durch die erste Ableitung der Funktion $E = f(x)$ der quantenchemischen Methode und erhalten wird, mit E=Gesamtenergie der Vorstufe für den Übergangszustand und x=Kernkoordinaten des Moleküls in der Vorstufe für den Übergangszustand,

C4.1 wenn die Gradient-Norm C3 $\nabla$ $0 \leq \nabla \leq 0{,}03$ $E_h a_0^{-1}$ beträgt, dann Fortführung des Verfahrens mit Schritt D1, oder

C4.2 wenn die Gradient-Norm C3 $\nabla > 0{,}03$ $E_h a_0^{-1}$ $E_h a_0^{-1}$ beträgt, dann Wiederholung der Schritte C1 bis C3 bis eine Gradient-Norm C3 $\nabla$ $0 \leq \nabla \leq 0{,}03$ $E_h a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Variation der Vorstufe aus Schritt C4.1 oder der Vorstufe aus Schritt B4.1, indem folgende Schritte für ein Atom innerhalb des in B1 definierten Funktionsraums durchgeführt werden

D1.1 Auswahl eines Atoms aus dem in Schritt B1 ausgewählten Funktionsraum,

D1.2 Auswahl eines Vektors für eine Auslenkung des in Schritt D1.1 gewählten Atoms

D1.3 Auslenkung des in Schritt D1.1 ausgewählten Atoms anhand des Vektors aus Schritt D1.2 aus seiner Position in der Vorstufe, wobei die Position einmal um einen positiven Wert des Betrags des Vektors und einmal um einen negativen Wert des Betrags des Vektors ausgelenkt wird, so dass bei der Durchführung der Schritte D1.1 bis D.1.3 zwei ausgelenkte Vorstufen erhalten werden,

D2 Geometrieoptimierung der zwei ausgelenkten Vorstufen aus Schritt D1 mittels der quantenchemischen Methode unter der Randbedingung dass die in D1 erhaltenen Bindungsabstände konstant gehalten werden, so dass zwei optimierte Vorstufen (i) und (ii) erhalten werden,

D3 Berechnung der Energie der zwei optimierten Vorstufen (i) und (ii) aus Schritt D2 mittels des quantenchemischen Verfahrens,

D4 Vergleich des Wertes der Energie der zwei optimierten Vorstufen mit dem Wert der Energie C3 oder B3 der Vorstufe, die in Schritt D1 verwendet wurde,

D4.1. wenn der Wert der Energie der optimierten Vorstufe (i) und der Wert der Energie der optimierten Vorstufe (ii) jeweils kleiner sind als der Wert der Energie C3 oder B3 der Vorstufe, die in Schritt D1 verwendet wurde, dann Einordnung der Vorstufe, die in Schritt D1 verwendet wurde, als Übergangszustand,

D4.2. wenn der Wert der Energie der optimierten Vorstufe (i) oder der Wert der Energie der optimierten Vorstufe (ii) nicht jeweils kleiner ist als der Wert der Energie C3 oder B3 der Vorstufe, die in Schritt D1 verwendet wurde, dann Wiederholung des Verfahrens ab Schritt C1.

In einer sechzehnten Ausführungsform betrifft die Erfindung ein computerlesbares Speichermedium, umfassend Befehle, die bei der Ausführung durch einen Computer diesen veranlassen, die folgenden Schritte eines Verfahrens durchzuführen

**A Erzeugen einer Startgeometrie**

A1 Bereitstellung der dreidimensionalen Darstellung mindestens eines Moleküls im energetischen Grundzustand,

A2 Auswahl von mindestens einer Bindung des mindestens einen Moleküls und Auswahl einer Länge der Bindung, wobei die ausgewählte Länge nicht der Länge der Bindung im energetischen Grundzustand des Moleküls entspricht, so dass eine Startgeometrie für die chemische Reaktion erhalten wird,
A3 Dreidimensionale Darstellung der Startgeometrie in kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst,

B2 Geometrieoptimierung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) der quantenchemischen Methode und erhalten wird, mit E=Gesamtenergie der optimierten Startgeometrie und x=Kernkoordinaten des Moleküls in der optimierten Startgeometrie,

B4.1 wenn die Gradient-Norm B3 $\nabla\, 0 \leq \nabla \leq 0{,}03\ E_h\ a_0^{-1}$ beträgt, dann Einordnung der optimierten Startgeometrie als eine Vorstufe für den Übergangszustand der chemischen Reaktion und Fortführung des Verfahrens mit Schritt D1, oder

B4.2 wenn die Gradient-Norm B3 $\nabla > 0{,}03\ E_h\ a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

**C Ermittlung der Vorstufe für den Übergangszustand der chemischen Reaktion**

C1 Variation der optimierten Startgeometrie unter Anwendung eines Monte-Carlo-Algorithmus wobei

C1.1 wenigstens ein Atom aus dem in Schritt B1 ausgewählten Funktionsraum zufällig ausgewählt wird,

C1.2 ein Vektor für eine Auslenkung des in Schritt C1 gewählten Atoms zufällig ausgewählt wird, wobei der zufällig ausgewählte Betrag des Vektors mit der Gradient-Norm B3 gewichtet wird,

C1.3 das in Schritt C1.1 ausgewählte Atom anhand des Vektors aus Schritt C1.2 aus der Position des Atoms in der optimierten Startgeometrie ausgelenkt wird, so dass eine Vorstufe für einen Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schritt A2 die Länge hat, die in Schritt C3 ermittelt wurde,

C3 Ermittlung der Gradient-Norm C3 für die Vorstufe für den Übergangszustand aus Schritt C2, wobei die Gradient-Norm durch die erste Ableitung der Funktion E = f(x) der quantenchemischen Methode und erhalten wird, mit E=Gesamtenergie der Vorstufe für den Übergangszustand und x=Kernkoordinaten des Moleküls in der Vorstufe für den Übergangszustand,

C4.1 wenn die Gradient-Norm C3 $\nabla\, 0 \leq \nabla \leq 0{,}03\ E_h\ a_0^{-1}$ beträgt, dann Fortführung des Verfahrens mit Schritt D1, oder

C4.2 wenn die Gradient-Norm C3 $\nabla > 0{,}03\ E_h\ a_0^{-1}\ E_h\ a_0^{-1}$ beträgt, dann Wiederholung der Schritte C1 bis C3 bis eine Gradient-Norm C3 $\nabla\, 0 \leq \nabla \leq 0{,}03\ E_h\ a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Variation der Vorstufe aus Schritt C4.1 oder der Vorstufe aus Schritt B4.1, indem folgende Schritte für ein Atom innerhalb des in B1 definierten Funktionsraums durchgeführt werden

D1.1 Auswahl eines Atoms aus dem in Schritt B1 ausgewählten Funktionsraum,

D1.2 Auswahl eines Vektors für eine Auslenkung des in Schritt D1.1 gewählten Atoms

D1.3 Auslenkung des in Schritt D1.1 ausgewählten Atoms anhand des Vektors aus Schritt D1.2 aus seiner Position in der Vorstufe, wobei die Position einmal um einen positiven Wert des Betrags des Vektors und einmal um einen negativen Wert des Betrags des Vektors ausgelenkt wird, so dass bei der Durchführung der Schritte D1.1 bis D.1.3 zwei ausgelenkte Vorstufen erhalten werden,

D2 Geometrieoptimierung der zwei ausgelenkten Vorstufen aus Schritt D1 mittels der quantenchemischen Methode unter der Randbedingung dass die in D1 erhaltenen Bindungsabstände konstant gehalten werden, so dass zwei optimierte Vorstufen (i) und (ii) erhalten werden,

D3 Berechnung der Energie der zwei optimierten Vorstufen (i) und (ii) aus Schritt D2 mittels des quantenchemischen Verfahrens,

D4 Vergleich des Wertes der Energie der zwei optimierten Vorstufen mit dem Wert der Energie C3 oder B3 der Vorstufe, die in Schritt D1 verwendet wurde,

D4.1. wenn der Wert der Energie der optimierten Vorstufe (i) und der Wert der Energie der optimierten Vorstufe (ii) jeweils kleiner sind als der Wert der Energie C3 oder B3 der Vorstufe, die in Schritt D1 verwendet wurde, dann Einordnung der Vorstufe, die in Schritt D1 verwendet wurde, als Übergangszustand,

D4.2. wenn der Wert der Energie der optimierten Vorstufe (i) oder der Wert der Energie der optimierten Vorstufe (ii) nicht jeweils kleiner ist als der Wert der Energie C3 oder B3 der Vorstufe, die in Schritt D1 verwendet wurde, dann Wiederholung des Verfahrens ab Schritt C1.

In einer siebzehnten Ausführungsform betrifft die Erfindung die Verwendung eines Computerprogramms nach Ausführungsform 15 oder eines computerlesbaren Speichermediums nach Ausführungsform 16 zur Bewertung von Übergangszuständen einer chemischen Reaktion, insbesondere einer Polymersynthese.

In einer achtzehnten Ausführungsform betrifft die Erfindung Verfahren gemäß einer der ersten bis dreizehnten Ausführungsform , dadurch gekennzeichnet, dass Information über den gemäß Schritt D.1 ermittelten Übergangszustand und/oder der gemäß Schritt D.2 ermittelten Gleichgewichtszustand einem Benutzer mitgeteilt wird.

In einer neunzehnten Ausführungsform betrifft die Erfindung Verfahren gemäß einer der ersten bis dreizehnten Ausführungsform, dadurch gekennzeichnet, dass Information über den gemäß Schritt D.1 ermittelten Übergangszustand und/oder der gemäß Schritt D.2 ermittelten Gleichgewichtszustand durch einen Benutzer empfangen wird.

In einer zwanzigsten Ausführungsform betrifft die Erfindung Verfahren gemäß einer der neunten bis dreizehnten Ausführungsform, dadurch gekennzeichnet, dass nach Schritt D.1 und/oder nach Schritt D.2 das Molekül I synthetisiert wird.

In einer einundzwanzigsten Ausführungsform betrifft die Erfindung Verfahren gemäß einer der neunten bis dreizehnten Ausführungsform, dadurch gekennzeichnet, dass nach Schritt D.1 und/oder nach Schritt D.2 unter eine chemische Reaktion mit dem Molekül I als Katalysator durchgeführt wird.

In einer zweiundzwanzigsten Ausführungsform betrifft die Erfindung Verfahren gemäß einer der neunten bis dreizehnten oder einundzwanzigsten Ausführungsform dadurch gekennzeichnet, dass nach Schritt D.1 und/oder nach Schritt D.2 unter eine chemische Reaktion mit dem Molekül II als Reaktionspartner durchgeführt wird.

Anhand der nachfolgenden Beispiele soll die Erfindung verdeutlicht werden, ohne jedoch darauf beschränkt zu werden.

Es wurde die Aktivität von verschiedenen Katalysatoren für die Reaktion eines endständige Isocyanat-Gruppen aufweisenden Prepolymers mit Wasser untersucht. Dazu wurden zunächst mit Hilfe des erfindungsgemäßen Verfahrens die Aktivierungsenergien für Reaktion mit dem jeweiligen Katalysator berechnet. Die berechneten Werte wurden dann mit den Ergebnissen von Laborversuchen der zuvor berechneten Reaktionen verglichen. Als Maßstab für die Aktivität der getesteten Katalysatoren wurde bei den Laborversuchen die Reaktionstemperatur herangezogen oberhalb derer die Katalysatoren Aktivität zeigten. Ob die Katalysatoren Aktivität zeigten wurde im Laborexperiment anhand der Freisetzung von Gasen und dem Aufschäumen der Reaktionsmischung festgestellt.

Die Laborversuche wurden wie folgt durchgeführt:

**Synthese eines HDI-Prepolymers mit endständigen Isocyanatgruppen**

Da handelsübliche Prepolymere Rückstande von Katalysatoren enthalten, die das Versuchsergebnis verfälschen könnten, wurde ein Prepolymer für die Durchführung der Versuche synthetisiert. Dazu wurden unter Argon-Gegenstrom 20,00 g (48,193 mmol; 1,0 Äquivalente) eines Polypropylenglykols (PET1004) mit einem mittleren Molekulargewicht von 415 g/mol vorgelegt und 16,212g Hexamethylendiisocyanat (2,0 Äquivalente) hinzu gegeben. Die Mischung wurde über einen Zeitraum von 3h unter Rühren auf 80°C erhitzt. Nach dem Abkühlen auf Raumtemperatur und einwöchiger Lagerung des Produkts bei Raumtemperatur wurde eine zähflüssige durchsichtige Substanz erhalten.

Das zahlengemittelte Molekulargewicht des HDI-Präpolymers wurde mit Hilfe von Gelpermeationschromatographie ermittelt. Dabei wurde die Probe in THF mit Polystyrol als Standard auf einer GPC-Säule vom Typ PSS SDV $5\mu$m linear S THF gemessen. Dabei wurde eine Molekularmasse von $M_n$=789,7 ermittelt.

(Die theoretischen Molekularmassen sind 751,4 g/mol bei einer 2-fachen Verlängerung des verwendeten Polypropylenglykols mit HDI und 583,2 g/mol bei einer einfachen Verlängerung des verwendeten Polypropylenglykols mit HDI).

**Testreaktionen**

Für die Reaktion des HDI-Prepolymers mit Wasser wurden jeweils die folgenden Katalysatoren verwendet:

- DBN: 1,5-Diazabicyclo[4,3,0]non-5-en;
- $NEt_3$: Triethylamin;
- DMA: Dimethylanilin.

In einem Reagenzglas mit Magnetrührer wurden 1,0 Äquivalente HDI-Präpolymer und 2,0 Äquivalente Wasser vorgelegt. Der Inhalt des Reagenzglas wurde gründlich durchmischt und unter Rühren 0,1 Äquivalente des jeweiligen Katalysators zugesetzt. Die Temperatur des Gemischs wurde mit Hilfe eines Ölbads mit Thermostat langsam erhöht, bis eine deutliche Gasentwicklung beobachtet werden konnte.

**Tabelle 1: Molmassen und Stoffmengen der eingesetzten Substanzen**

| Komponente | M [g/mol] | n [mol] | m [mg] | Äq | d [g/m l] | V [ml] |
|---|---|---|---|---|---|---|
| HDI-Präpolymer | 751,4[1)] | 5,857 | 4401 | 1,0 | - | - |
| $H_2O$ | 18,015 | 11,720 | 211 | 2,0 | 1,00 | 0,21 |
| DBN | 124,18 | 0,586 | 73 | 0,1 | 1,01 | 0,07 |
| $NEt_3$ | 101,19 | 0,586 | 59 | 0,1 | 0,73 | 0,08 |
| DMA | 121,18 | 0,586 | 71 | 0,1 | 0,96 | 0,07 |
| 1) Theoretisches Molekulargewicht für ein Präpolymer aus zwei Äquivalenten HDI und einem Äquivalent des verwendeten Polypropylenglykols. | | | | | | |

Durchführung der quantenmechanischen Berechnungen

Die quantenmechanischen Berechnungen wurden mit dem Programmpaket Turbomole durchgeführt. Die verwendete Dichtefunktionaltheoretische Methode war das TPSS-Dichtefunktional mit einem def2-SVP-Basissatz, so wie standardmäßig im Turbomole-Programmpaket implementiert. Dazu wurde als Computer ein Intel Xeon E5-2667v4 mit 16 Core-Prozessoren mit einer Taktfrequenz von 3.20GHz und 25MB Zwischenspeicher mit einem RAM Speicher von 128 GB DDR4 2400 rg ECC. Die Berechnungen wurden auf jeweils zwei Prozessoren ausgeführt wobei ein Speichervolumen von 8000 MB zur Verfügung gestellt wurde.

Pro untersuchter Reaktion wurde eine Startgeometrie als Ausgangspunkt für die Ermittlung eines Übergangszustandes gewählt. Der Zeitaufwand für die Berechnung der jeweiligen Aktivierungsenergien betrug bis zu 36 Stunden.

Zur Simulation der Aktivierungsenergien wurde die Reaktion eines Prepolymers enthaltend zwei endständige, HDI-basierte Isocyanatgruppen und ein syntaktisches Polypropylenglykol mit sieben Wiederholungseinheiten basierend auf Propylenoxid, bestehen aus insgesamt 121 Atomen, einem Molekül Wasser und dem jeweiligen Katalysator betrachtet (insgesamt zwischen 144 und 146 Atomen). Zur Generierung der Startgeometrien der Ausführungsbeispiele in Tabelle 3 wurde im Beispiel von DBN, gemäß Verfahrensschritt A1, ein Molekül des Prepolymers, ein Molekül des DBN-Katalysators und ein Molekül Wasser mit einem geeigneten Programm zur Visualisierung dreidimensionaler Molekülstrukturen gezeichnet. Die Position des Kohlenstoffatoms der betrachteten Isocyanatgruppe wurde gemäß Verfahrensschritt A2 in einen Abstand von 175,5 pm zu dem Sauerstoffatom des betrachteten Wassermoleküls gebracht und eines der Wasserstoffatome im Wassermolekül wurde in einen Abstand von 117,4 pm zum Sauerstoff des Wassermoleküls gebracht. Die so erzeugte Molekülgeometrie wurde gemäß Verfahrensschritt A3 in einer Darstellung kartesischer Koordinaten gespeichert und die an den so angeordneten Bindungsabständen beteiligten Atome zur Definition des Funktionsraumes gemäß Verfahrensschritt B1 verwendet.

Mit der erfindungsgemäßen Methode konnten die in **Tabelle 2** dargestellten Aktivierungsenergien erhalten werden.

Mit Hilfe der im Stand der Technik bekannten Pseudo-Newton-Raphson-basierten Methode zur Optimierung von Übergangszuständen konnte unter gleichen Bedingungen, also mit denselben Startgeometrien für den Übergangszustand, in keinem der gezeigten Fälle ein Übergangszustand und damit eine Aktivierungsenergie erhalten werden.

**Tabelle 2: Zusammenstellung experimenteller und berechneter Aktivierungsenergien**

| Katalysator | Aktivierungsenergie berechnet [Kcal/mol] | Temperatur bei der eine Aktivität des Katalysators im Laborversuch zu beobachten war [°C] | Gradientnorm der optimierten Startgeometrie [$E_h$ $a_0^{-1}$] | Gradientnorm des Übergangszustands [$E_h$ $a_0^{-1}$] |
|---|---|---|---|---|
| DBN | 13,2 | 23 | 0.024810 | 0.009638 |
| NEt$_3$ | 16,8 | 40 | 0.019049 | 0.006762 |
| DMA | 20,4 | 110-174[1)] | 0.029652 | 0.008176 |
| 1) sehr unscharf zu bestimmender Temperaturpunkt. Reaktion nur schwach erkennbar. | | | | |

Die Daten aus Tabelle 2 zeigen, dass mit Hilfe der erfindungsgemäßen Methode die Molekülgeometrie eines Übergangszustands einer chemischen Reaktion mit sehr wenig Benutzeraufwand und so präzise berechnet werden kann, dass anhand der berechneten Aktivierungsenergie eine zutreffende Aussage über die Kinetik der chemischen Reaktion getroffen werden kann.

Das zahlengemittelte Molekulargewichts des HDI-Präpolymers, das in den Laborversuchen eingesetzt wurde, liegt im gleichen Bereich wie die theoretisch berechnete Masse eines Polypropylenglykols, das 2-fach mit HDI verlängert wurde. Die Laborversuche ähneln somit den Reaktionsbedingungen, die für die Simulation der Reaktion zur Berechnung der Aktivierungsenergie herangezogen wurden und bilden die Simulation realistisch ab.

Die auf der Grundlage des erfindungsgemäßen Verfahrens berechnete Aktivierungsenergie für DBN war etwas geringer als für NEt$_3$ und deutlich niedriger als diejenige für DMA. Aus den berechneten Ergebnissen kann der Schluss gezogen werden, dass DBN als Katalysator bei einer Reaktion des HDI-Prepolymers mit Wasser einen Übergangszustand ermöglicht der eine niedrigere Energie aufweist als bei der Verwendung von NEt$_3$ oder DMA als Katalysator. Folglich müsste bei der Durchführung der Reaktion mit DBN als Katalysator weniger oder keine Energie (ausgehend von Raumtemperatur) zugeführt werden, damit die Reaktion abläuft und Produkte erhalten werden.

Diese Schlüsse aus den berechneten Aktivierungsenergien spiegeln sich in den Ergebnissen der Laborversuche wieder. Während die Reaktion des HDI-Prepolymers mit Wasser und DBN als Katalysator bereits bei 23 °C ablief, musste bei der Verwendung von NEt$_3$ als Katalysator die Reaktionsmischung auf 40 °C erhöht werden. Aus den berechneten Aktivierungsenergien konnte bereits der Schluss gezogen werden, dass für die Durchführung der Reaktion mit NEt$_3$ eine höhere Aktivierungsenergie erforderlich sein würde.

Das gleiche gilt für die Verwendung von DMA als Katalysator. Hier war die berechnete Aktivierungsenergie deutlich höher als bei NEt$_3$ als Katalysator. Dieses Ergebnis konnte durch die Laborversuche verifiziert werden. Bei der Verwendung NEt$_3$ als Katalysator konnte erst bei einer Temperatur von mehr als 100 °C eine Reaktion erkannt werden und dies auch nur schwach.

**Patentansprüche**

**1.** Computerimplementiertes Verfahren zur Berechnung von Übergangszuständen einer chemischen Reaktion umfassend die Schritte

**A Erzeugen einer Startgeometrie**

A1 Bereitstellung der dreidimensionalen Darstellung mindestens eines Moleküls im energetischen Grundzustand,
A2 Auswahl von mindestens einer Bindung des mindestens einen Moleküls und Auswahl einer Länge der Bindung, wobei die ausgewählte Länge nicht der Länge der Bindung im energetischen Grundzustand des Moleküls entspricht, so dass eine Startgeometrie für die chemische Reaktion erhalten wird,
A3 Dreidimensionale Darstellung der Startgeometrie in kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst,
B2 Geometrieoptimierung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) der quantenchemischen Methode und erhalten wird, mit E=Gesamtenergie der optimierten Startgeometrie und x=Kernkoordinaten des Moleküls in der optimierten Startgeometrie,

B4.1 wenn die Gradient-Norm B3 $\nabla$ $0 \leq \nabla \leq 0{,}03$ $E_h$ $a_0^{-1}$ beträgt, dann Einordnung der optimierten Startgeometrie als eine Vorstufe für den Übergangszustand der chemischen Reaktion und Fortführung des Verfahrens mit Schritt D1, oder

B4.2 wenn die Gradient-Norm B3 $\nabla > 0{,}03$ $E_h$ $a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

## C Ermittlung der Vorstufe für den Übergangszustand der chemischen Reaktion

C1 Variation der optimierten Startgeometrie unter Anwendung eines Monte-Carlo-Algorithmus wobei

C1.1 wenigstens ein Atom aus dem in Schritt B1 ausgewählten Funktionsraum zufällig ausgewählt wird,

C1.2 ein Vektor für eine Auslenkung des in Schritt C1 gewählten Atoms zufällig ausgewählt wird, wobei der zufällig ausgewählte Betrag des Vektors mit der Gradient-Norm B3 gewichtet wird,

C1.3 das in Schritt C1.1 ausgewählte Atom anhand des Vektors aus Schritt C1.2 aus der Position des Atoms in der optimierten Startgeometrie ausgelenkt wird, so dass eine Vorstufe für einen Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schritt A2 die Länge hat, die in Schritt C1.3 ermittelt wurde,

C3 Ermittlung der Gradient-Norm C3 für die Vorstufe für den Übergangszustand aus Schritt C2, wobei die Gradient-Norm durch die erste Ableitung der Funktion E = f(x) der quantenchemischen Methode und erhalten wird, mit E=Gesamtenergie der Vorstufe für den Übergangszustand und x=Kernkoordinaten des Moleküls in der Vorstufe für den Übergangszustand,

C4.1 wenn die Gradient-Norm C3 $\nabla$ $0 \leq \nabla \leq 0{,}03$ $E_h$ $a_0^{-1}$ beträgt, dann Fortführung des Verfahrens mit Schritt D1, oder

C4.2 wenn die Gradient-Norm C3 $\nabla > 0{,}03$ $E_h$ $a_0^{-1}$ beträgt, dann Wiederholung der Schritte C1 bis C3 bis eine Gradient-Norm C3 $\nabla$ $0 \leq \nabla \leq 0{,}03$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorangegangenen Wiederholungen variiert wird, welche im Vergleich zu allen bisher erhaltenen Gradient-Normen C3 und B3 den niedrigsten Wert für die Gradient-Norm C3 oder B3 aufweist,

## D Ermittlung des Übergangszustandes

D1 Variation der Vorstufe aus Schritt C4.1 oder der Vorstufe aus Schritt B4.1, indem folgende Schritte für ein Atom innerhalb des in B1 definierten Funktionsraums durchgeführt werden

D1.1 Auswahl eines Atoms aus dem in Schritt B1 ausgewählten Funktionsraum,

D1.2 Auswahl eines Vektors für eine Auslenkung des in Schritt D1.1 gewählten Atoms,

D1.3 Auslenkung des in Schritt D1.1 ausgewählten Atoms anhand des Vektors aus Schritt D1.2 aus seiner Position in der Vorstufe, wobei die Position einmal um einen positiven Wert des Betrags des Vektors und einmal um einen negativen Wert des Betrags des Vektors ausgelenkt wird, so dass bei der Durchführung der Schritte D1.1 bis D.1.3 zwei ausgelenkte Vorstufen erhalten werden,

D2 Geometrieoptimierung der zwei ausgelenkten Vorstufen aus Schritt D1 mittels der quantenchemischen Methode unter der Randbedingung dass die in D1 erhaltenen Bindungsabstände konstant gehalten werden, so dass zwei optimierte Vorstufen (i) und (ii) erhalten werden,

D3 Berechnung der Energie der zwei optimierten Vorstufen (i) und (ii) aus Schritt D2 mittels des quantenchemischen Verfahrens,

D4 Vergleich des Wertes der Energie der zwei optimierten Vorstufen mit dem Wert der Gradient-Norm C3 oder B3 der Vorstufe, die in Schritt D1 verwendet wurde,

D4.1. wenn der Wert der Energie der optimierten Vorstufe (i) und der Wert der Energie der optimierten Vorstufe (ii) jeweils kleiner sind als der Wert der Energie C3 oder B3 der Vorstufe, die in Schritt D1 verwendet wurde, dann Einordnung der Vorstufe, die in Schritt D1 verwendet wurde, als Übergangszustand,

D4.2. wenn der Wert der Energie der optimierten Vorstufe (i) oder der Wert der Energie der optimierten Vorstufe (ii) nicht jeweils kleiner ist als der Wert der Energie C3 oder B3 der Vorstufe, die in Schritt D1 verwendet wurde, dann Wiederholung des Verfahrens ab Schritt C1.

2. Verfahren nach Anspruch 2, **dadurch gekennzeichnet, dass** die quantenchemische Methode aus Schritten **B2, B3, C2, C3, D2** und **D3** eine semiempirische Methode, Dichte-Funktional-theoretische Methode oder eine Annäherung der Schrödinger-Gleichung ist, insbesondere ist die quantenchemische Methode aus Schritten **B2, B3, C2, C3, D2** und **D3** eine Dichte-Funktional-theoretische Methode.

3. Verfahren nach Anspruch 1 oder 2, **dadurch gekennzeichnet, dass** die chemische Reaktion eine Synthese ist ausgewählt aus der Gruppe bestehend aus Polymersynthesen, insbesondere Polyurethansynthesen, Synthesen von Monomeren für Polymerisationsreaktionen, industriell benötigte Grundchemikalien Plattformchemikalien, Additiven , Tensiden und pharmakologischen Wirkstoffen.

4. Verfahren nach einem der vorstehenden Ansprüche, **dadurch gekennzeichnet, dass** in Schritt **A1** mindestens zwei Moleküle I und II bereitgestellt werden und in Schritt **A2** alternativ oder zusätzlich zu der mindestens einen Bindung auch mindestens ein Abstand zwischen mindestens einem Atom aus Molekül I und mindestens einem Atom aus Molekül II sowie die Länge des mindestens einen Abstands ausgewählt werden, wobei die Länge des Abstands insbesondere höchstens 230 pm beträgt .

5. Verfahren nach Anspruch 4, **dadurch gekennzeichnet, dass** Molekül I ein Katalysator für die chemische Reaktion ist, insbesondere für eine Polymersynthese, und Molekül II ein Edukt der chemischen Reaktion, wobei Molekül I bevorzugt eine Größe von 2 bis 1000 Atomen aufweisen soll und Molekül II bevorzugt eine Größe von 2 bis 1000 Atomen aufweisen soll, insbesondere soll die Summe der Atome aus Molekül I und aus Molekül II mehr als 100 Atome betragen.

6. Verfahren gemäß einem der Ansprüche 1 bis 5, **dadurch gekennzeichnet, dass** Information über den gemäß Schritt D.1 ermittelten Übergangszustand und/oder der gemäß Schritt D.2 ermittelten Gleichgewichtszustand einem Benutzer mitgeteilt wird.

7. Verfahren gemäß einem der Ansprüche 1 bis 5, **dadurch gekennzeichnet, dass** Information über den gemäß Schritt D.1 ermittelten Übergangszustand und/oder der gemäß Schritt D.2 ermittelten Gleichgewichtszustand durch einen Benutzer empfangen wird.

8. Verfahren gemäß Anspruch 5, **dadurch gekennzeichnet, dass** nach Schritt D.1 und/oder nach Schritt D.2 das Molekül I synthetisiert wird.

9. Verfahren gemäß Anspruch 5, **dadurch gekennzeichnet, dass** nach Schritt D.1 und/oder nach Schritt D.2 unter eine chemische Reaktion mit dem Molekül I als Katalysator durchgeführt wird.

10. Verfahren gemäß Anspruch 5 oder 9, **dadurch gekennzeichnet, dass** nach Schritt D.1 und/oder nach Schritt D.2 unter eine chemische Reaktion mit dem Molekül II als Reaktionspartner durchgeführt wird.

11. System zur Datenverarbeitung umfassend Mittel zur Ausführung eines Verfahrens umfassend die Schritte

**A Erzeugen einer Startgeometrie**

A1 Bereitstellung der dreidimensionalen Darstellung mindestens eines Moleküls im energetischen Grundzustand,

A2 Auswahl von mindestens einer Bindung des mindestens einen Moleküls und Auswahl einer Länge der Bindung, wobei die ausgewählte Länge nicht der Länge der Bindung im energetischen Grundzustand des Moleküls entspricht, so dass eine Startgeometrie für die chemische Reaktion erhalten wird,

A3 Dreidimensionale Darstellung der Startgeometrie in kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst,

B2 Geometrieoptimierung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) der quantenchemischen Methode und erhalten wird, mit E=Gesamtenergie der optimierten Startgeometrie und x=Kernkoordinaten des Moleküls in der optimierten Startgeometrie,

B4.1 wenn die Gradient-Norm B3 $\nabla$ $0 \leq \nabla \leq 0,03$ $E_h$ $a_0^{-1}$ beträgt, dann Einordnung der optimierten Startgeometrie als eine Vorstufe für den Übergangszustand der chemischen Reaktion und Fortführung des Verfahrens mit Schritt D1, oder

B4.2 wenn die Gradient-Norm B3 $\nabla$ > 0,03 $E_h$ $a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

## C Ermittlung der Vorstufe für den Übergangszustand der chemischen Reaktion

C1 Variation der optimierten Startgeometrie unter Anwendung eines Monte-Carlo-Algorithmus wobei

C1.1 wenigstens ein Atom aus dem in Schritt B1 ausgewählten Funktionsraum zufällig ausgewählt wird,

C1.2 ein Vektor für eine Auslenkung des in Schritt C1 gewählten Atoms zufällig ausgewählt wird, wobei der zufällig ausgewählte Betrag des Vektors mit der Gradient-Norm B3 gewichtet wird,

C1.3 das in Schritt C1.1 ausgewählte Atom anhand des Vektors aus Schritt C1.2 aus der Position des Atoms in der optimierten Startgeometrie ausgelenkt wird, so dass eine Vorstufe für einen Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schritt A2 die Länge hat, die in Schritt C1.3 ermittelt wurde,

C3 Ermittlung der Gradient-Norm C3 für die Vorstufe für den Übergangszustand aus Schritt C2, wobei die Gradient-Norm durch die erste Ableitung der Funktion E = f(x) der quantenchemischen Methode und erhalten wird, mit E=Gesamtenergie der Vorstufe für den Übergangszustand und x=Kernkoordinaten des Moleküls in der Vorstufe für den Übergangszustand,

C4.1 wenn die Gradient-Norm C3 $\nabla$ $0 \leq \nabla \leq 0,03$ $E_h$ $a_0^{-1}$ beträgt, dann Fortführung des Verfahrens mit Schritt D1, oder

C4.2 wenn die Gradient-Norm C3 $\nabla$ > 0,03 $E_h$ $a_0^{-1}$ $E_h$ $a_0^{-1}$ beträgt, dann Wiederholung der Schritte C1 bis C3 bis eine Gradient-Norm C3 $\nabla$ $0 \leq \nabla \leq 0,03$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorangegangen Wiederholungen variiert wird, welche im Vergleich zu allen bisher erhaltenen Gradient-Normen C3 und B3 den niedrigsten Wert für die Gradient-Norm C3 oder B3 aufweist,

## D Ermittlung des Übergangszustandes

D1 Variation der Vorstufe aus Schritt C4.1 oder der Vorstufe aus Schritt B4.1, indem folgende Schritte für ein Atom innerhalb des in B1 definierten Funktionsraums durchgeführt werden

D1.1 Auswahl eines Atoms aus dem in Schritt B1 ausgewählten Funktionsraum,

D1.2 Auswahl eines Vektors für eine Auslenkung des in Schritt D1.1 gewählten Atoms

D1.3 Auslenkung des in Schritt D1.1 ausgewählten Atoms anhand des Vektors aus Schritt D1.2 aus seiner Position in der Vorstufe, wobei die Position einmal um einen positiven Wert des Betrags des Vektors und einmal um einen negativen Wert des Betrags des Vektors ausgelenkt wird, so dass bei der Durchführung der Schritte D1.1 bis D.1.3 zwei ausgelenkte Vorstufen erhalten werden,

D2 Geometrieoptimierung der zwei ausgelenkten Vorstufen aus Schritt D1 mittels der quantenchemischen Methode unter der Randbedingung dass die in D1 erhaltenen Bindungsabstände konstant gehalten werden, so dass zwei optimierte Vorstufen (i) und (ii) erhalten werden,

D3 Berechnung der Energie der zwei optimierten Vorstufen (i) und (ii) aus Schritt D2 mittels des quantenchemischen Verfahrens,

D4 Vergleich des Wertes der Energie der zwei optimierten Vorstufen mit dem Wert der Energie C3 oder B3 der Vorstufe, die in Schritt D1 verwendet wurde,

D4.1. wenn der Wert der Energie der optimierten Vorstufe (i) und der Wert der Energie der optimierten Vorstufe (ii) jeweils kleiner sind als der Wert der Energie C3 oder B3 der Vorstufe, die in Schritt D1 verwendet wurde, dann Einordnung der Vorstufe, die in Schritt D1 verwendet wurde, als Übergangszustand,

D4.2. wenn der Wert der Energie der optimierten Vorstufe (i) oder der Wert der Energie der optimierten Vorstufe (ii) nicht jeweils kleiner ist als der Wert der Energie C3 oder B3 der Vorstufe, die in Schritt D1 verwendet wurde, dann Wiederholung des Verfahrens ab Schritt C1.

12. Computerprogramm, umfassend Befehle, die bei der Ausführung des Programms durch einen Computer diesen veranlassen, die folgenden Schritte eines Verfahrens durchzuführen

**A Erzeugen einer Startgeometrie**

A1 Bereitstellung der dreidimensionalen Darstellung mindestens eines Moleküls im energetischen Grundzustand,

A2 Auswahl von mindestens einer Bindung des mindestens einen Moleküls und Auswahl einer Länge der Bindung, wobei die ausgewählte Länge nicht der Länge der Bindung im energetischen Grundzustand des Moleküls entspricht, so dass eine Startgeometrie für die chemische Reaktion erhalten wird,

A3 Dreidimensionale Darstellung der Startgeometrie in kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst,

B2 Geometrieoptimierung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) der quantenchemischen Methode und erhalten wird, mit E=Gesamtenergie der optimierten Startgeometrie und x=Kernkoordinaten des Moleküls in der optimierten Startgeometrie,

B4.1 wenn die Gradient-Norm B3 $\nabla$ $0 \leq \nabla \leq 0,03$ $E_h$ $a_0^{-1}$ beträgt, dann Einordnung der optimierten Startgeometrie als eine Vorstufe für den Übergangszustand der chemischen Reaktion und Fortführung des Verfahrens mit Schritt D1, oder

B4.2 wenn die Gradient-Norm B3 $\nabla$ $> 0,03$ $E_h$ $a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

**C Ermittlung der Vorstufe für den Übergangszustand der chemischen Reaktion**

C1 Variation der optimierten Startgeometrie unter Anwendung eines Monte-Carlo-Algorithmus wobei

C1.1 wenigstens ein Atom aus dem in Schritt B1 ausgewählten Funktionsraum zufällig ausgewählt wird,

C1.2 ein Vektor für eine Auslenkung des in Schritt C1 gewählten Atoms zufällig ausgewählt wird, wobei der zufällig ausgewählte Betrag des Vektors mit der Gradient-Norm B3 gewichtet wird,

C1.3 das in Schritt C1.1 ausgewählte Atom anhand des Vektors aus Schritt C1.2 aus der Position des Atoms in der optimierten Startgeometrie ausgelenkt wird, so dass eine Vorstufe für einen Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schritt A2 die Länge hat, die in Schritt C1.3 ermittelt wurde,

C3 Ermittlung der Gradient-Norm C3 für die Vorstufe für den Übergangszustand aus Schritt C2, wobei die Gradient-Norm durch die erste Ableitung der Funktion E = f(x) der quantenchemischen Methode und erhalten wird, mit E=Gesamtenergie der Vorstufe für den Übergangszustand und x=Kernkoordinaten des Moleküls in der Vorstufe für den Übergangszustand,

C4.1 wenn die Gradient-Norm C3 $\nabla$ $0 \leq \nabla \leq 0,03$ $E_h$ $a_0^{-1}$ beträgt, dann Fortführung des Verfahrens mit Schritt D1, oder

C4.2 wenn die Gradient-Norm C3 $\nabla$ > 0,03 $E_h\,a_0^{-1}$ $E_h\,a_0^{-1}$ beträgt, dann Wiederholung der Schritte C1 bis C3 bis eine Gradient-Norm C3 $\nabla$ 0 $\leq \nabla \leq$ 0,03 $E_h\,a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorangegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Variation der Vorstufe aus Schritt C4.1 oder der Vorstufe aus Schritt B4.1, indem folgende Schritte für ein Atom innerhalb des in B1 definierten Funktionsraums durchgeführt werden

D1.1 Auswahl eines Atoms aus dem in Schritt B1 ausgewählten Funktionsraum,

D1.2 Auswahl eines Vektors für eine Auslenkung des in Schritt D1.1 gewählten Atoms

D1.3 Auslenkung des in Schritt D1.1 ausgewählten Atoms anhand des Vektors aus Schritt D1.2 aus seiner Position in der Vorstufe, wobei die Position einmal um einen positiven Wert des Betrags des Vektors und einmal um einen negativen Wert des Betrags des Vektors ausgelenkt wird, so dass bei der Durchführung der Schritte D1.1 bis D.1.3 zwei ausgelenkte Vorstufen erhalten werden,

D2 Geometrieoptimierung der zwei ausgelenkten Vorstufen aus Schritt D1 mittels der quantenchemischen Methode unter der Randbedingung dass die in D1 erhaltenen Bindungsabstände konstant gehalten werden, so dass zwei optimierte Vorstufen (i) und (ii) erhalten werden,

D3 Berechnung der Energie der zwei optimierten Vorstufen (i) und (ii) aus Schritt D2 mittels des quantenchemischen Verfahrens,

D4 Vergleich des Wertes der Energie der zwei optimierten Vorstufen mit dem Wert der Energie C3 oder B3 der Vorstufe, die in Schritt D1 verwendet wurde,

D4.1. wenn der Wert der Energie der optimierten Vorstufe (i) und der Wert der Energie der optimierten Vorstufe (ii) jeweils kleiner sind als der Wert der Energie C3 oder B3 der Vorstufe, die in Schritt D1 verwendet wurde, dann Einordnung der Vorstufe, die in Schritt D1 verwendet wurde, als Übergangszustand,

D4.2. wenn der Wert der Energie der optimierten Vorstufe (i) oder der Wert der Energie der optimierten Vorstufe (ii) nicht jeweils kleiner ist als der Wert der Energie C3 oder B3 der Vorstufe, die in Schritt D1 verwendet wurde, dann Wiederholung des Verfahrens ab Schritt C1.

13. Computerlesbares Speichermedium, umfassend Befehle, die bei der Ausführung durch einen Computer diesen veranlassen, die folgenden Schritte eines Verfahrens durchzuführen

**A Erzeugen einer Startgeometrie**

A1 Bereitstellung der dreidimensionalen Darstellung mindestens eines Moleküls im energetischen Grundzustand,

A2 Auswahl von mindestens einer Bindung des mindestens einen Moleküls und Auswahl einer Länge der Bindung, wobei die ausgewählte Länge nicht der Länge der Bindung im energetischen Grundzustand des Moleküls entspricht, so dass eine Startgeometrie für die chemische Reaktion erhalten wird,

A3 Dreidimensionale Darstellung der Startgeometrie in kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst,

B2 Geometrieoptimierung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) der quantenchemischen Methode und erhalten wird, mit E=Gesamtenergie der optimierten Startgeometrie und x=Kernkoordinaten des Moleküls in der optimierten Startgeometrie,

B4.1wenn die Gradient-Norm B3 $\nabla$ $0 \leq \nabla \leq 0,03$ $E_h$ $a_0^{-1}$ beträgt, dann Einordnung der optimierten Startgeometrie als eine Vorstufe für den Übergangszustand der chemischen Reaktion und Fortführung des Verfahrens mit Schritt D1, oder

B4.2wenn die Gradient-Norm B3 $\nabla$ $> 0,03$ $E_h$ $a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

## C Ermittlung der Vorstufe für den Übergangszustand der chemischen Reaktion

C1 Variation der optimierten Startgeometrie unter Anwendung eines Monte-Carlo-Algorithmus wobei

C1.1wenigstens ein Atom aus dem in Schritt B1 ausgewählten Funktionsraum zufällig ausgewählt wird,

C1.2 ein Vektor für eine Auslenkung des in Schritt C1 gewählten Atoms zufällig ausgewählt wird, wobei der zufällig ausgewählte Betrag des Vektors mit der Gradient-Norm B3 gewichtet wird,

C1.3 das in Schritt C1.1 ausgewählte Atom anhand des Vektors aus Schritt C1.2 aus der Position des Atoms in der optimierten Startgeometrie ausgelenkt wird, so dass eine Vorstufe für einen Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schritt A2 die Länge hat, die in Schritt C1.3 ermittelt wurde,

C3 Ermittlung der Gradient-Norm C3 für die Vorstufe für den Übergangszustand aus Schritt C2, wobei die Gradient-Norm durch die erste Ableitung der Funktion E = f(x) der quantenchemischen Methode und erhalten wird, mit E=Gesamtenergie der Vorstufe für den Übergangszustand und x=Kernkoordinaten des Moleküls in der Vorstufe für den Übergangszustand,

C4.1wenn die Gradient-Norm C3 $\nabla$ $0 \leq \nabla \leq 0,03$ $E_h$ $a_0^{-1}$ beträgt, dann Fortführung des Verfahrens mit Schritt D1, oder

C4.2wenn die Gradient-Norm C3 $\nabla$ $> 0,03$ $E_h$ $a_0^{-1}$ $E_h$ $a_0^{-1}$ beträgt, dann Wiederholung der Schritte C1 bis C3 bis eine Gradient-Norm C3 $\nabla$ $0 \leq \nabla \leq 0,03$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorangegangen Wiederholungen variiert wird, welche im Vergleich zu allen bisher erhaltenen Gradient-Normen C3 und B3 den niedrigsten Wert für die Gradient-Norm C3 oder B3 aufweist,

## D Ermittlung des Übergangszustandes

D1 Variation der Vorstufe aus Schritt C4.1 oder der Vorstufe aus Schritt B4.1, indem folgende Schritte für ein Atom innerhalb des in B1 definierten Funktionsraums durchgeführt werden

D1.1 Auswahl eines Atoms aus dem in Schritt B1 ausgewählten Funktionsraum,

D1.2 Auswahl eines Vektors für eine Auslenkung des in Schritt D1.1 gewählten Atoms

D1.3 Auslenkung des in Schritt D1.1 ausgewählten Atoms anhand des Vektors aus Schritt D1.2 aus seiner Position in der Vorstufe, wobei die Position einmal um einen positiven Wert des Betrags des Vektors und einmal um einen negativen Wert des Betrags des Vektors ausgelenkt wird, so dass bei der Durchführung der Schritte D1.1 bis D.1.3 zwei ausgelenkte Vorstufen erhalten werden,

D2 Geometrieoptimierung der zwei ausgelenkten Vorstufen aus Schritt D1 mittels der quantenchemischen Methode unter der Randbedingung dass die in D1 erhaltenen Bindungsabstände konstant gehalten werden, so dass zwei optimierte Vorstufen (i) und (ii) erhalten werden,

D3 Berechnung der Energie der zwei optimierten Vorstufen (i) und (ii) aus Schritt D2 mittels des quantenchemischen Verfahrens,

D4 Vergleich des Wertes der Energie der zwei optimierten Vorstufen mit dem Wert der Energie C3 oder B3 der Vorstufe, die in Schritt D1 verwendet wurde,

D4.1. wenn der Wert der Energie der optimierten Vorstufe (i) und der Wert der Energie der optimierten Vorstufe (ii) jeweils kleiner sind als der Wert der Energie C3 oder B3 der Vorstufe, die in Schritt D1 verwendet wurde, dann Einordnung der Vorstufe, die in Schritt D1 verwendet wurde, als Übergangszustand,

D4.2. wenn der Wert der Energie der optimierten Vorstufe (i) oder der Wert der Energie der optimierten Vorstufe (ii) nicht jeweils kleiner ist als der Wert der Energie C3 oder B3 der Vorstufe, die in Schritt D1 verwendet wurde, dann Wiederholung des Verfahrens ab Schritt C1.

14. Verwendung eines Computerprogramms nach Anspruch 12 oder eines computerlesbaren Speichermediums nach Anspruch 13 zur Bewertung von Übergangszuständen einer chemischen Reaktion, insbesondere einer Polymersynthese.

**Claims**

1. Computer-implemented method of calculating transition states of a chemical reaction, comprising the steps of:

**A generating a starting geometry**

A1 providing the three-dimensional representation of at least one molecule at ground state energy,
A2 selecting at least one bond of the at least one molecule and selecting a length of the bond, where the selected length does not correspond to the length of the bond at ground state energy of the molecule, such that a starting geometry for the chemical reaction is obtained,
A3 representing the starting geometry in three dimensions in Cartesian and/or internal coordinates,

**B ascertaining an optimized starting geometry**

B1 defining a function space encompassing the at least one bond from step A2 and the atoms joined by this bond,
B2 optimizing the geometry of the function space selected in step B1 by means of a quantum-chemical method and with the boundary condition that the length of the at least one bond selected in step A2 is kept constant, such that an optimized starting geometry is obtained,
B3 ascertaining the gradient norm B3 for the optimized starting geometry, where the gradient norm is obtained via the first derivative of a function $E = f(x)$ of the quantum-chemical method, with E = total energy of the optimized starting geometry and x = nuclear coordinates of the molecule in the optimized starting geometry,
B4.1 when the gradient norm B3 V is $0 \leq V \leq 0.03\ E_h\ a_0^{-1}$, classifying the optimized starting geometry as a precursor to the transition state of the chemical reaction and continuing the method with step D1, or
B4.2 when the gradient norm B3 V is $> 0.03\ E_h\ a_0^{-1}$, ascertaining the precursor to the transition state of the chemical reaction proceeding from the optimized starting geometry by a method comprising the following steps:

**C ascertaining the precursor to the transition state of the chemical reaction**

C1 varying the optimized starting geometry using a Monte Carlo algorithm, wherein
C1.1 at least one atom from the function space selected in step B1 is selected at random,
C1.2 a vector for a deflection of the atom chosen in step C1 is selected at random, weighting the randomly chosen magnitude of the vector by the gradient norm B3,
C1.3 the atom selected in step C1.1 is deflected from the position of the atom in the optimized starting geometry using the vector from step C1.2, so as to obtain a precursor to a transition state of the chemical reaction,
C2 optimizing the geometry of the precursor to the transition state by means of the quantum-chemical method and with the boundary condition that the at least one bond from step A2 has the length that was ascertained in step C1.3,
C3 ascertaining the gradient norm C3 for the precursor to the transition state from step C2, where the gradient norm is obtained via the first derivative of the function $E = f(x)$ of the quantum-chemical method, with E = total energy of the precursor to the transition state and x = nuclear coordinates of the molecule in the precursor to the transition state,
C4.1 when the gradient norm C3 V is $0 \leq V \leq 0.03\ E_h\ a_0^{-1}$, continuing the method with step D1, or
C4.2 when the gradient norm C3 V $> 0.03\ E_h\ a_0^{-1}$, repeating steps C1 to C3 until a gradient norm C3 V of $0 \leq V \leq 0.03\ E_h\ a_0^{-1}$ is obtained, wherein

(a) if steps C1 to C3 have been performed once, the geometry of the precursor to the transition state is varied in step C1 when the value of its gradient norm C3 is lower than the value of the gradient norm B3 of the optimized starting geometry, or

(b) if steps C1 to C3 have been performed more than once, the geometry of the optimized starting geometry or that precursor to the transition state from the preceding repetitions that has the lowest value for the gradient norm C3 or B3 compared to all the gradient norms C3 and B3 previously obtained is varied in step C1,

**D ascertaining the transition state**

D1 varying the precursor from step C4.1 or the precursor from step B4.1 by performing the following steps for one atom within the function space defined in B1:

D1.1 selecting an atom from the function space selected in step B1,
D1.2 selecting a vector for a deflection of the atom chosen in step D1.1,
D1.3 deflecting the atom selected in step D1.1 using the vector from step D1.2 from its position in the precursor, wherein the position is deflected once by the positive value of the magnitude of the vector and once by the negative value of the magnitude of the vector, such that two deflected precursors are obtained when steps D1.1 to D1.3 are conducted,

D2 optimizing the geometry of the two deflected precursors from step D1 by means of the quantum-chemical method under the constraint that the bond distances obtained in D1 are kept constant, such that two optimized precursors (i) and (ii) are obtained,
D3 calculating the energy of the two optimized precursors (i) and (ii) from step D2 by means of the quantum-chemical method,
D4 comparing the energy values of the two optimized precursors with the value of the gradient norm C3 or B3 of the precursor that was used in step D1,
D4.1. if the energy value of the optimized precursor (i) and the energy value of the optimized precursor (ii) are each smaller than the energy value C3 or B3 of the precursor that was used in step D1, classifying the precursor that was used in step D1 as the transition state,
D4.2. if the energy value of the optimized precursor (i) or the energy value of the optimized precursor (ii) is not smaller than the energy value C3 or B3 of the precursor that was used in step D1, repeating the method from step C1.

2. Method according to Claim 2, **characterized in that** the quantum-chemical method from steps B2, B3, C2, C3, D2 and D3 is a semiempirical method, density functional theory method or an approximation of the Schrödinger equation, the quantum-chemical method from steps B2, B3, C2, C3, D2 and D3 especially being a density functional theory method.

3. Method according to Claim 1 or 2, **characterized in that** the chemical reaction is a synthesis selected from the group consisting of polymer syntheses, especially polyurethane syntheses, syntheses of monomers for polymerization reactions, industrially required commodity chemicals, platform chemicals, additives, surfactants and active pharmacological ingredients.

4. Method according to any of the preceding claims, **characterized in that**, in step A1, at least two molecules I and II are provided and, in step A2, alternatively or additionally to the at least one bond, at least one distance between at least one atom from molecule I and at least one atom from molecule II and the length of the at least one distance is also selected, where the length of the distance is especially not more than 230 pm.

5. Method according to Claim 4, **characterized in that** molecule I is a catalyst for the chemical reaction, especially for a polymer synthesis, and molecule II is a reactant in the chemical reaction, where molecule I is preferably to have a size of 2 to 1000 atoms and molecules II is preferably to have a size of 2 to 1000 atoms, where the sum total of the atoms from molecule I and from molecule II is especially to be more than 100 atoms.

6. Method according to any of Claims 1 to 5, **characterized in that** information as to the transition state ascertained in step D.1 and/or the equilibrium state ascertained in step D.2 is communicated to a user.

7. Method according to any of Claims 1 to 5, **characterized in that** information as to the transition state ascertained in step D.1 and/or the equilibrium state ascertained in step D.2 is received by a user.

8. Method according to Claim 5, **characterized in that** the molecule I is synthesized after step D.1 and/or after step D.2.

9. Method according to Claim 5, **characterized in that** after step D.1 and/or after step D.2, a chemical reaction is performed with molecule I as catalyst.

10. Method according to Claim 5 or 9, **characterized in that** after step D.1 and/or after step D.2, a chemical reaction is performed with molecule II as co-reactant.

11. System for data processing, comprising means of executing a method comprising the steps of:

**A generating a starting geometry**

A1 providing the three-dimensional representation of at least one molecule at ground state energy,
A2 selecting at least one bond of the at least one molecule and selecting a length of the bond, where the selected length does not correspond to the length of the bond at ground state energy of the molecule, such that a starting geometry for the chemical reaction is obtained,
A3 representing the starting geometry in three dimensions in Cartesian and/or internal coordinates,

**B ascertaining an optimized starting geometry**

B1 defining a function space encompassing the at least one bond from step A2 and the atoms joined by this bond,
B2 optimizing the geometry of the function space selected in step B1 by means of a quantum-chemical method and with the boundary condition that the length of the at least one bond selected in step A2 is kept constant, such that an optimized starting geometry is obtained,
B3 ascertaining the gradient norm B3 for the optimized starting geometry, where the gradient norm is obtained via the first derivative of a function $E = f(x)$ of the quantum-chemical method, with $E$ = total energy of the optimized starting geometry and $x$ = nuclear coordinates of the molecule in the optimized starting geometry,
B4.1 when the gradient norm B3 V is $0 \leq V \leq 0.03\ E_h\ a_0^{-1}$, classifying the optimized starting geometry as a precursor to the transition state of the chemical reaction and continuing the method with step D1, or
B4.2 when the gradient norm B3 V is $> 0.03\ E_h\ a_0^{-1}$, ascertaining the precursor to the transition state of the chemical reaction proceeding from the optimized starting geometry by a method comprising the following steps:

**C ascertaining the precursor to the transition state of the chemical reaction**

C1 varying the optimized starting geometry using a Monte Carlo algorithm, wherein
C1.1 at least one atom from the function space selected in step B1 is selected at random,
C1.2 a vector for a deflection of the atom chosen in step C1 is selected at random, weighting the randomly chosen magnitude of the vector by the gradient norm B3,
C1.3 the atom selected in step C1.1 is deflected from the position of the atom in the optimized starting geometry using the vector from step C1.2, so as to obtain a precursor to a transition state of the chemical reaction,
C2 optimizing the geometry of the precursor to the transition state by means of the quantum-chemical method and with the boundary condition that the at least one bond from step A2 has the length that was ascertained in step C1.3,
C3 ascertaining the gradient norm C3 for the precursor to the transition state from step C2, where the gradient norm is obtained via the first derivative of the function $E = f(x)$ of the quantum-chemical method, with $E$ = total energy of the precursor to the transition state and $x$ = nuclear coordinates of the molecule in the precursor to the transition state,
C4.1 when the gradient norm C3 V is $0 \leq V \leq 0.03\ E_h\ a_0^{-1}$, continuing the method with step D1, or
C4.2 when the gradient norm C3 V $> 0.03\ E_h\ a_0^{-1}\ E_h\ a_0^{-1}$, repeating steps C1 to C3 until a gradient norm C3 V of $0 \leq V \leq 0.03\ E_h\ a_0^{-1}$ is obtained, wherein

(a) if steps C1 to C3 have been performed once, the geometry of the precursor to the transition state is varied in step C1 when the value of its gradient norm C3 is lower than the value of the gradient norm B3 of the optimized starting geometry, or

(b) if steps C1 to C3 have been performed more than once, the geometry of the optimized starting geometry or that precursor to the transition state from the preceding repetitions that has the lowest value for the gradient norm C3 or B3 compared to all the gradient norms C3 and B3 previously obtained is varied in step C1,

**D ascertaining the transition state**

D1 varying the precursor from step C4.1 or the precursor from step B4.1 by performing the following steps for one atom within the function space defined in B1:

D1.1 selecting an atom from the function space selected in step B1,
D1.2 selecting a vector for a deflection of the atom chosen in step D1.1,
D1.3 deflecting the atom selected in step D1.1 using the vector from step D1.2 from its position in the precursor, wherein the position is deflected once by the positive value of the magnitude of the vector and once by the negative value of the magnitude of the vector, such that two deflected precursors are obtained when steps D1.1 to D1.3 are conducted,

D2 optimizing the geometry of the two deflected precursors from step D1 by means of the quantum-chemical method under the constraint that the bond distances obtained in D1 are kept constant, such that two optimized precursors (i) and (ii) are obtained,
D3 calculating the energy of the two optimized precursors (i) and (ii) from step D2 by means of the quantum-chemical method,
D4 comparing the energy values of the two optimized precursors with the energy value C3 or B3 of the precursor that was used in step D1,
D4.1. if the energy value of the optimized precursor (i) and the energy value of the optimized precursor (ii) are each smaller than the energy value C3 or B3 of the precursor that was used in step D1, classifying the precursor that was used in step D1 as the transition state,
D4.2. if the energy value of the optimized precursor (i) or the energy value of the optimized precursor (ii) is not smaller than the energy value C3 or B3 of the precursor that was used in step D1, repeating the method from step C1.

12. Computer program comprising commands that, on execution of the program by a computer, cause it to perform the following steps of a method:

**A generating a starting geometry**

A1 providing the three-dimensional representation of at least one molecule at ground state energy,
A2 selecting at least one bond of the at least one molecule and selecting a length of the bond, where the selected length does not correspond to the length of the bond at ground state energy of the molecule, such that a starting geometry for the chemical reaction is obtained,
A3 representing the starting geometry in three dimensions in Cartesian and/or internal coordinates,

**B ascertaining an optimized starting geometry**

B1 defining a function space encompassing the at least one bond from step A2 and the atoms joined by this bond,
B2 optimizing the geometry of the function space selected in step B1 by means of a quantum-chemical method and with the boundary condition that the length of the at least one bond selected in step A2 is kept constant, such that an optimized starting geometry is obtained,
B3 ascertaining the gradient norm B3 for the optimized starting geometry, where the gradient norm is obtained via the first derivative of a function $E = f(x)$ of the quantum-chemical method, with E = total energy of the optimized starting geometry and x = nuclear coordinates of the molecule in the optimized starting geometry,
B4.1 when the gradient norm B3 V is $0 \leq V \leq 0.03\ E_h\ a_0^{-1}$, classifying the optimized starting geometry as a precursor to the transition state of the chemical reaction and continuing the method with step D1, or
B4.2 when the gradient norm B3 V is $> 0.03\ E_h\ a_0^{-1}$, ascertaining the precursor to the transition state of the chemical reaction proceeding from the optimized starting geometry by a method comprising the following steps:

**C ascertaining the precursor to the transition state of the chemical reaction**

C1 varying the optimized starting geometry using a Monte Carlo algorithm, wherein

C1.1 at least one atom from the function space selected in step B1 is selected at random,

C1.2 a vector for a deflection of the atom chosen in step C1 is selected at random, weighting the randomly chosen magnitude of the vector by the gradient norm B3,

C1.3 the atom selected in step C1.1 is deflected from the position of the atom in the optimized starting geometry using the vector from step C1.2, so as to obtain a precursor to a transition state of the chemical reaction,

C2 optimizing the geometry of the precursor to the transition state by means of the quantum-chemical method and with the boundary condition that the at least one bond from step A2 has the length that was ascertained in step C1.3,

C3 ascertaining the gradient norm C3 for the precursor to the transition state from step C2, where the gradient norm is obtained via the first derivative of the function $E = f(x)$ of the quantum-chemical method, with E = total energy of the precursor to the transition state and x = nuclear coordinates of the molecule in the precursor to the transition state,

C4.1 when the gradient norm C3 V is $0 \leq V \leq 0.03\ E_h\ a_0^{-1}$, continuing the method with step D1, or

C4.2 when the gradient norm C3 V > $0.03\ E_h\ a_0^{-1}$, repeating steps C1 to C3 until a gradient norm C3 V of $0 \leq V \leq 0.03\ E_h\ a_0^{-1}$ is obtained, wherein

(a) if steps C1 to C3 have been performed once, the geometry of the precursor to the transition state is varied in step C1 when the value of its gradient norm C3 is lower than the value of the gradient norm B3 of the optimized starting geometry, or

(b) if steps C1 to C3 have been performed more than once, the geometry of the optimized starting geometry or that precursor to the transition state from the preceding repetitions that has the lowest value for the gradient norm C3 or B3 compared to all the gradient norms C3 and B3 previously obtained is varied in step C1,

**D ascertaining the transition state**

D1 varying the precursor from step C4.1 or the precursor from step B4.1 by performing the following steps for one atom within the function space defined in B1:

D1.1 selecting an atom from the function space selected in step B1,

D1.2 selecting a vector for a deflection of the atom chosen in step D1.1,

D1.3 deflecting the atom selected in step D1.1 using the vector from step D1.2 from its position in the precursor, wherein the position is deflected once by the positive value of the magnitude of the vector and once by the negative value of the magnitude of the vector, such that two deflected precursors are obtained when steps D1.1 to D1.3 are conducted,

D2 optimizing the geometry of the two deflected precursors from step D1 by means of the quantum-chemical method under the constraint that the bond distances obtained in D1 are kept constant, such that two optimized precursors (i) and (ii) are obtained,

D3 calculating the energy of the two optimized precursors (i) and (ii) from step D2 by means of the quantum-chemical method,

D4 comparing the energy values of the two optimized precursors with the energy value C3 or B3 of the precursor that was used in step D1,

D4.1. if the energy value of the optimized precursor (i) and the energy value of the optimized precursor (ii) are each smaller than the energy value C3 or B3 of the precursor that was used in step D1, classifying the precursor that was used in step D1 as the transition state,

D4.2. if the energy value of the optimized precursor (i) or the energy value of the optimized precursor (ii) is not smaller than the energy value C3 or B3 of the precursor that was used in step D1, repeating the method from step C1.

13. Computer-readable storage medium comprising commands that, on execution by a computer, cause it to perform the following steps of a method:

**A generating a starting geometry**

A1 providing the three-dimensional representation of at least one molecule at ground state energy,

A2 selecting at least one bond of the at least one molecule and selecting a length of the bond, where the selected length does not correspond to the length of the bond at ground state energy of the molecule, such that a starting geometry for the chemical reaction is obtained,

A3 representing the starting geometry in three dimensions in Cartesian and/or internal coordinates,

**B ascertaining an optimized starting geometry**

B1 defining a function space encompassing the at least one bond from step A2 and the atoms joined by this bond,

B2 optimizing the geometry of the function space selected in step B1 by means of a quantum-chemical method and with the boundary condition that the length of the at least one bond selected in step A2 is kept constant, such that an optimized starting geometry is obtained,

B3 ascertaining the gradient norm B3 for the optimized starting geometry, where the gradient norm is obtained via the first derivative of a function $E = f(x)$ of the quantum-chemical method, with E = total energy of the optimized starting geometry and x = nuclear coordinates of the molecule in the optimized starting geometry,

B4.1 when the gradient norm B3 V is $0 \leq V \leq 0.03\ E_h\ a_0^{-1}$, classifying the optimized starting geometry as a precursor to the transition state of the chemical reaction and continuing the method with step D1, or

B4.2 when the gradient norm B3 V is $> 0.03\ E_h\ a_0^{-1}$, ascertaining the precursor to the transition state of the chemical reaction proceeding from the optimized starting geometry by a method comprising the following steps:

**C ascertaining the precursor to the transition state of the chemical reaction**

C1 varying the optimized starting geometry using a Monte Carlo algorithm, wherein

C1.1 at least one atom from the function space selected in step B1 is selected at random,

C1.2 a vector for a deflection of the atom chosen in step C1 is selected at random, weighting the randomly chosen magnitude of the vector by the gradient norm B3,

C1.3 the atom selected in step C1.1 is deflected from the position of the atom in the optimized starting geometry using the vector from step C1.2, so as to obtain a precursor to a transition state of the chemical reaction,

C2 optimizing the geometry of the precursor to the transition state by means of the quantum-chemical method and with the boundary condition that the at least one bond from step A2 has the length that was ascertained in step C1.3,

C3 ascertaining the gradient norm C3 for the precursor to the transition state from step C2, where the gradient norm is obtained via the first derivative of the function $E = f(x)$ of the quantum-chemical method, with E = total energy of the precursor to the transition state and x = nuclear coordinates of the molecule in the precursor to the transition state,

C4.1 when the gradient norm C3 V is $0 \leq V \leq 0.03\ E_h\ a_0^{-1}$, continuing the method with step D1, or

C4.2 when the gradient norm C3 $V > 0.03\ E_h\ a_0^{-1}\ E_h\ a_0^{-1}$, repeating steps C1 to C3 until a gradient norm C3 V of $0 \leq V \leq 0.03\ E_h\ a_0^{-1}$ is obtained, wherein

(a) if steps C1 to C3 have been performed once, the geometry of the precursor to the transition state is varied in step C1 when the value of its gradient norm C3 is lower than the value of the gradient norm B3 of the optimized starting geometry, or

(b) if steps C1 to C3 have been performed more than once, the geometry of the optimized starting geometry or that precursor to the transition state from the preceding repetitions that has the lowest value for the gradient norm C3 or B3 compared to all the gradient norms C3 and B3 previously obtained is varied in step C1,

**D ascertaining the transition state**

D1 varying the precursor from step C4.1 or the precursor from step B4.1 by performing the following steps for one atom within the function space defined in B1:

D1.1 selecting an atom from the function space selected in step B1,

D1.2 selecting a vector for a deflection of the atom chosen in step D1.1,

D1.3 deflecting the atom selected in step D1.1 using the vector from step D1.2 from its position in the precursor, wherein the position is deflected once by the positive value of the magnitude of the vector and once by the negative value of the magnitude of the vector, such that two deflected precursors are obtained when steps D1.1 to D1.3 are conducted,

D2 optimizing the geometry of the two deflected precursors from step D1 by means of the quantum-chemical method under the constraint that the bond distances obtained in D1 are kept constant, such that two optimized precursors (i) and (ii) are obtained,
D3 calculating the energy of the two optimized precursors (i) and (ii) from step D2 by means of the quantum-chemical method,
D4 comparing the energy values of the two optimized precursors with the energy value C3 or B3 of the precursor that was used in step D1,
D4.1. if the energy value of the optimized precursor (i) and the energy value of the optimized precursor (ii) are each smaller than the energy value C3 or B3 of the precursor that was used in step D1, classifying the precursor that was used in step D1 as the transition state,
D4.2. if the energy value of the optimized precursor (i) or the energy value of the optimized precursor (ii) is not smaller than the energy value C3 or B3 of the precursor that was used in step D1, repeating the method from step C1.

14. Use of a computer program according to Claim 12 or of a computer-readable storage medium according to Claim 13 for evaluating transition states of a chemical reaction, especially a polymer synthesis.

**Revendications**

1. Procédé mis en œuvre par un ordinateur pour le calcul d'états de transition d'une réaction chimique, comprenant les étapes

A génération d'une géométrie de départ
A1 mise à disposition de la représentation tridimensionnelle d'au moins une molécule dans l'état énergétique de base,
A2 sélection d'au moins une liaison de ladite au moins une molécule et sélection d'une longueur de la liaison, la longueur sélectionnée ne correspondant pas à la longueur de la liaison dans l'état énergétique de base de la molécule, de telle sorte qu'une géométrie de départ pour la réaction chimique est obtenue,
A3 représentation tridimensionnelle de la géométrie de départ en coordonnées cartésiennes et/ou internes,
B détermination d'une géométrie de départ optimisée
B1 définition d'un espace fonctionnel qui comprend ladite au moins une liaison de l'étape A2 et les atomes reliés par cette liaison,
B2 optimisation géométrique de l'espace fonctionnel sélectionné dans l'étape B1 au moyen d'une méthode de chimie quantique et sous la condition limite selon laquelle la longueur sélectionnée dans l'étape A2 de ladite au moins une liaison est maintenue constante, de telle sorte qu'une géométrie de départ optimisée est obtenue,
B3 détermination de la norme de gradient B3 pour la géométrie de départ optimisée, la norme de gradient étant obtenue par la première dérivée d'une fonction $E = f(x)$ de la méthode de chimie quantique, avec $E$ = énergie totale de la géométrie de départ optimisée et $x$ = les coordonnées de noyau de la molécule dans la géométrie de départ optimisée,
B4.1 lorsque la norme de gradient B3 V répond à $0 \leq \nabla \leq 0,03\ E_h\ a_0^{-1}$, alors classification de la géométrie de départ optimisée comme un stade préliminaire à l'état de transition de la réaction chimique et poursuite du procédé par l'étape D1 ou
B4.2 lorsque la norme de gradient B3 V répond à $>0,03\ E_h\ a_0^{-1}$, alors détermination du stade préliminaire à l'état de transition de la réaction chimique partant de la géométrie de départ optimisée par un procédé comprenant les étapes suivantes :

C détermination du stade préliminaire à l'état de transition de la réaction chimique
C1 variation de la géométrie de départ optimisée à l'aide d'un algorithme de Monte-Carlo
C1.1 au moins un atome de l'espace fonctionnel sélectionné dans l'étape B1 étant sélectionné aléatoirement,
C1.2 un vecteur pour une déviation de l'atome sélectionné dans l'étape C1 étant sélectionné aléatoirement, la longueur sélectionnée aléatoirement du vecteur étant pondérée par la norme de gradient B3,

C1.3 l'atome sélectionné dans l'étape C1.1 étant dévié à l'aide du vecteur de l'étape C1.2 à partir de la position de l'atome dans la géométrie de départ optimisée de telle sorte qu'un stade préliminaire à un état de transition de la réaction chimique est obtenu,

C2 optimisation géométrique du stade préliminaire à l'état de transition au moyen de la méthode de chimie quantique et sous la condition limite selon laquelle ladite au moins une liaison de l'étape A2 présente la longueur qui a été déterminée dans l'étape C1.3,

C3 détermination de la norme de gradient C3 pour le stade préliminaire à l'état de transition de l'étape C2, la norme de gradient étant obtenue par la première dérivée d'une fonction E = f(x) de la méthode de chimie quantique, avec E = énergie totale du stade préliminaire à l'état de transition et x = les coordonnées de noyau de la molécule dans le stade préliminaire à l'état de transition,

C4.1 lorsque la norme de gradient C3 V répond à $0 \leq \nabla \leq 0,03\ E_h\ a_0^{-1}$, alors poursuite du procédé par l'étape D1 ou

C4.2 lorsque la norme de gradient C3 V répond à $> 0,03\ E_h\ a_0^{-1}$, alors répétition des étapes C1 à C3 jusqu'à ce qu'une norme de gradient C3 V répondant à $0 \leq \nabla \leq 0,03\ E_h\ a_0^{-1}$ soit obtenue

> (a) pour le cas où les étapes C1 à C3 ont été effectuées une fois, dans l'étape C1, la géométrie du stade préliminaire à l'état de transition étant modifiée lorsque la valeur de sa norme de gradient C3 est inférieure à la valeur de la norme de gradient B3 de la géométrie de départ optimisée ou
>
> (b) pour le cas où les étapes C1 à C3 ont été effectuées plus d'une fois, dans l'étape C1, la géométrie de la géométrie de départ optimisée ou du stade préliminaire respectif à l'état de transition, provenant des répétitions précédentes, qui présente la valeur la plus basse pour la norme de gradient C3 ou B3 par rapport à toutes les normes de gradient C3 et B3 obtenues jusqu'à présent, étant modifiée,

D détermination de l'état de transition

D1 variation du stade préliminaire de l'étape C4.1 ou du stade préliminaire de l'étape B4.1, en ce que les étapes suivantes pour un atome dans l'espace fonctionnel défini dans B1 sont effectuées

D1.1 sélection d'un atome de l'espace fonctionnel sélectionné dans l'étape B1,

D1.2 sélection d'un vecteur pour une déviation de l'atome sélectionné dans l'étape D1.1,

D1.3 déviation de l'atome sélectionné dans l'étape D1.1 de sa position dans le stade préliminaire à l'aide du vecteur de l'étape D1.2, la position étant déviée une fois d'une valeur positive de la longueur du vecteur et une fois d'une valeur négative de la longueur du vecteur, de telle sorte que lorsque les étapes D1.1 à D.1.3 sont effectuées, on obtient deux stades préliminaires déviés,

D2 optimisation géométrique des deux stades préliminaires déviés de l'étape D1 au moyen de la méthode de chimie quantique sous la condition limite selon laquelle les distances de liaison obtenues dans D1 sont maintenues constantes, de telle sorte que deux stades préliminaires optimisés (i) et (ii) sont obtenus,

D3 calcul de l'énergie des deux stades préliminaires optimisés (i) et (ii) de l'étape D2 au moyen de la méthode de chimie quantique,

D4 comparaison de la valeur énergétique des deux stades préliminaires optimisés à la valeur de la norme de gradient C3 ou B3 du stade préliminaire qui a été utilisé dans l'étape D1,

D4.1. lorsque la valeur énergétique du stade préliminaire optimisé (i) et la valeur énergétique du stade préliminaire optimisé (ii) sont respectivement inférieures à la valeur énergétique C3 ou B3 du stade préliminaire qui a été utilisé dans l'étape D1, alors classification du stade préliminaire qui a été utilisé dans l'étape D1 comme état de transition,

D4.2. lorsque la valeur énergétique du stade préliminaire optimisé (i) ou la valeur énergétique du stade préliminaire optimisé (ii) ne sont pas respectivement inférieures à la valeur énergétique C3 ou B3 du stade préliminaire qui a été utilisé dans l'étape D1, alors répétition du procédé à partir de l'étape C1.

**2.** Procédé selon la revendication 2, **caractérisé en ce que** la méthode de chimie quantique des étapes B2, B3, C2, C3, D2 et D3 est une méthode semi-empirique, une méthode de la théorie de la fonctionnelle de la densité ou une approche de l'équation de Schrödinger, en particulier, la méthode de chimie quantique des étapes B2, B3, C2, C3, D2 et D3 est une méthode de la théorie de la fonctionnelle de la densité.

**3.** Procédé selon la revendication 1 ou 2, **caractérisé en ce que** la réaction chimique est une synthèse choisie dans le groupe constitué par les synthèses de polymères, en particulier les synthèses de polyuréthanes, les synthèses de monomères pour des réactions de polymérisation, des produits chimiques de base nécessaires dans l'industrie, des produits chimiques de plateforme, des additifs, des tensioactifs et des substances actives pharmacologiques.

**4.** Procédé selon l'une des revendications précédentes, **caractérisé en ce que**, dans l'étape A1, au moins deux molécules I et II sont mises à disposition et, dans l'étape A2, en variante ou en plus de ladite au moins une liaison, on sélectionne également au moins une distance entre au moins un atome de la molécule I et au moins un atome de la molécule II ainsi que la longueur de ladite au moins une distance, la longueur de la distance valant en particulier au plus 230 pm.

**5.** Procédé selon la revendication 4, **caractérisé en ce que** la molécule I est un catalyseur pour la réaction chimique, en particulier pour une synthèse de polymères, et la molécule II est un produit de départ de la réaction chimique, la molécule I présentant de préférence une dimension de 2 à 1000 atomes et la molécule II présentant de préférence une dimension de 2 à 1000 atomes, en particulier, la somme des atomes de la molécule I et de la molécule II étant supérieure à 100 atomes.

**6.** Procédé selon l'une des revendications 1 à 5, **caractérisé en ce que** l'information concernant l'état de transition déterminé selon l'étape D.1 et/ou l'état d'équilibre déterminé selon l'étape D.2 est communiquée à un utilisateur.

**7.** Procédé selon l'une des revendications 1 à 5, **caractérisé en ce que** l'information concernant l'état de transition déterminé selon l'étape D.1 et/ou l'état d'équilibre déterminé selon l'étape D.2 est reçue par un utilisateur.

**8.** Procédé selon la revendication 5, **caractérisé en ce que** la molécule I est synthétisée après l'étape D.1 et/ou après l'étape D.2.

**9.** Procédé selon la revendication 5, **caractérisé en ce qu'**après l'étape D.1 et/ou après l'étape D.2, une réaction chimique est effectuée avec la molécule I en tant que catalyseur.

**10.** Procédé selon la revendication 5 ou 9, **caractérisé en ce qu'**après l'étape D.1 et/ou après l'étape D.2, une réaction chimique est effectuée avec la molécule II en tant que partenaire de réaction.

**11.** Système de traitement de données comprenant des moyens d'exécution d'un procédé comprenant les étapes

A génération d'une géométrie de départ
A1 mise à disposition de la représentation tridimensionnelle d'au moins une molécule dans l'état énergétique de base,
A2 sélection d'au moins une liaison de ladite au moins une molécule et sélection d'une longueur de la liaison, la longueur sélectionnée ne correspondant pas à la longueur de la liaison dans l'état énergétique de base de la molécule, de telle sorte qu'une géométrie de départ pour la réaction chimique est obtenue,
A3 représentation tridimensionnelle de la géométrie de départ en coordonnées cartésiennes et/ou internes,
B détermination d'une géométrie de départ optimisée
B1 définition d'un espace fonctionnel qui comprend ladite au moins une liaison de l'étape A2 et les atomes reliés par cette liaison,
B2 optimisation géométrique de l'espace fonctionnel sélectionné dans l'étape B1 au moyen d'une méthode de chimie quantique et sous la condition limite selon laquelle la longueur sélectionnée dans l'étape A2 de ladite au moins une liaison est maintenue constante, de telle sorte qu'une géométrie de départ optimisée est obtenue,
B3 détermination de la norme de gradient B3 pour la géométrie de départ optimisée, la norme de gradient étant obtenue par la première dérivée d'une fonction E = f(x) de la méthode de chimie quantique, avec E = énergie totale de la géométrie de départ optimisée et x = les coordonnées de noyau de la molécule dans la géométrie de départ optimisée,
B4.1 lorsque la norme de gradient B3 V répond à $0 \leq \nabla \leq 0,03\ E_h\ a_0^{-1}$, alors classification de la géométrie de départ optimisée comme un stade préliminaire à l'état de transition de la réaction chimique et poursuite du procédé par l'étape D1 ou
B4.2 lorsque la norme de gradient B3 répond à $V > 0,03\ E_h\ a_0^{-1}$, alors détermination du stade préliminaire à l'état de transition de la réaction chimique partant de la géométrie de départ optimisée par un procédé comprenant les étapes suivantes :

C détermination du stade préliminaire à l'état de transition de la réaction chimique
C1 variation de la géométrie de départ optimisée à l'aide d'un algorithme de Monte-Carlo
C1.1 au moins un atome de l'espace fonctionnel sélectionné dans l'étape B1 étant sélectionné aléatoirement,
C1.2 un vecteur pour une déviation de l'atome sélectionné dans l'étape C1 étant sélectionné aléatoirement, la longueur sélectionnée aléatoirement du vecteur étant pondérée par la norme de gradient B3,

C1.3 l'atome sélectionné dans l'étape C1.1 étant dévié à l'aide du vecteur de l'étape C1.2 à partir de la position de l'atome dans la géométrie de départ optimisée de telle sorte qu'un stade préliminaire à un état de transition de la réaction chimique est obtenu,

C2 optimisation géométrique du stade préliminaire à l'état de transition au moyen de la méthode de chimie quantique et sous la condition limite selon laquelle ladite au moins une liaison de l'étape A2 présente la longueur qui a été déterminée dans l'étape C1.3,

C3 détermination de la norme de gradient C3 pour le stade préliminaire à l'état de transition de l'étape C2, la norme de gradient étant obtenue par la première dérivée d'une fonction E = f(x) de la méthode de chimie quantique, avec E = énergie totale du stade préliminaire à l'état de transition et x = les coordonnées de noyau de la molécule dans le stade préliminaire à l'état de transition,

C4.1 lorsque la norme de gradient C3 V répond à $0 \leq \nabla \leq 0{,}03\ E_h\ a_0^{-1}$, alors poursuite du procédé par l'étape D1 ou

C4.2 lorsque la norme de gradient C3 V répond à $> 0{,}03\ E_h\ a_0^{-1}$, alors répétition des étapes C1 à C3 jusqu'à ce qu'une norme de gradient C3 V répondant à $0 \leq \nabla \leq 0{,}03\ E_h\ a_0^{-1}$ soit obtenue

(a) pour le cas où les étapes C1 à C3 ont été effectuées une fois, dans l'étape C1, la géométrie du stade préliminaire à l'état de transition étant modifiée lorsque la valeur de sa norme de gradient C3 est inférieure à la valeur de la norme de gradient B3 de la géométrie de départ optimisée ou

(b) pour le cas où les étapes C1 à C3 ont été effectuées plus d'une fois, dans l'étape C1, la géométrie de la géométrie de départ optimisée ou du stade préliminaire respectif à l'état de transition, provenant des répétitions précédentes, qui présente la valeur la plus basse pour la norme de gradient C3 ou B3 par rapport à toutes les normes de gradient C3 et B3 obtenues jusqu'à présent, étant modifiée,

D détermination de l'état de transition

D1 variation du stade préliminaire de l'étape C4.1 ou du stade préliminaire de l'étape B4.1, en ce que les étapes suivantes pour un atome dans l'espace fonctionnel défini dans B1 sont effectuées

D1.1 sélection d'un atome de l'espace fonctionnel sélectionné dans l'étape B1,

D1.2 sélection d'un vecteur pour une déviation de l'atome sélectionné dans l'étape D1.1,

D1.3 déviation de l'atome sélectionné dans l'étape D1.1 de sa position dans le stade préliminaire à l'aide du vecteur de l'étape D1.2, la position étant déviée une fois d'une valeur positive de la longueur du vecteur et une fois d'une valeur négative de la longueur du vecteur, de telle sorte que lorsque les étapes D1.1 à D.1.3 sont effectuées, on obtient deux stades préliminaires déviés,

D2 optimisation géométrique des deux stades préliminaires déviés de l'étape D1 au moyen de la méthode de chimie quantique sous la condition limite selon laquelle les distances de liaison obtenues dans D1 sont maintenues constantes, de telle sorte que deux stades préliminaires optimisés (i) et (ii) sont obtenus,

D3 calcul de l'énergie des deux stades préliminaires optimisés (i) et (ii) de l'étape D2 au moyen de la méthode de chimie quantique,

D4 comparaison de la valeur énergétique des deux stades préliminaires optimisés à la valeur énergétique C3 ou B3 du stade préliminaire qui a été utilisé dans l'étape D1,

D4.1. lorsque la valeur énergétique du stade préliminaire optimisé (i) et la valeur énergétique du stade préliminaire optimisé (ii) sont respectivement inférieures à la valeur énergétique C3 ou B3 du stade préliminaire qui a été utilisé dans l'étape D1, alors classification du stade préliminaire qui a été utilisé dans l'étape D1 comme état de transition,

D4.2. lorsque la valeur énergétique du stade préliminaire optimisé (i) ou la valeur énergétique du stade préliminaire optimisé (ii) ne sont pas respectivement inférieures à la valeur énergétique C3 ou B3 du stade préliminaire qui a été utilisé dans l'étape D1, alors répétition du procédé à partir de l'étape C1.

**12.** Programme informatique comprenant des instructions qui permettent de mettre en œuvre les étapes suivantes d'un procédé lors de l'exécution du programme par un ordinateur

A génération d'une géométrie de départ

A1 mise à disposition de la représentation tridimensionnelle d'au moins une molécule dans l'état énergétique de base,

A2 sélection d'au moins une liaison de ladite au moins une molécule et sélection d'une longueur de la liaison, la longueur sélectionnée ne correspondant pas à la longueur de la liaison dans l'état énergétique de base de la molécule, de telle sorte qu'une géométrie de départ pour la réaction chimique est obtenue,

A3 représentation tridimensionnelle de la géométrie de départ en coordonnées cartésiennes et/ou internes,

B détermination d'une géométrie de départ optimisée B1 définition d'un espace fonctionnel qui comprend ladite au moins une liaison de l'étape A2 et les atomes reliés par cette liaison,

B2 optimisation géométrique de l'espace fonctionnel sélectionné dans l'étape B1 au moyen d'une méthode de chimie quantique et sous la condition limite selon laquelle la longueur sélectionnée dans l'étape A2 de ladite au moins une liaison est maintenue constante, de telle sorte qu'une géométrie de départ optimisée est obtenue,

B3 détermination de la norme de gradient B3 pour la géométrie de départ optimisée, la norme de gradient étant obtenue par la première dérivée d'une fonction $E = f(x)$ de la méthode de chimie quantique, avec $E$ = énergie totale de la géométrie de départ optimisée et $x$ = les coordonnées de noyau de la molécule dans la géométrie de départ optimisée,

B4.1 lorsque la norme de gradient B3 V répond à $0 \leq \nabla \leq 0{,}03\ E_h\ a_0^{-1}$, alors classification de la géométrie de départ optimisée comme un stade préliminaire à l'état de transition de la réaction chimique et poursuite du procédé par l'étape D1 ou

B4.2 lorsque la norme de gradient B3 répond à $V > 0{,}03\ E_h\ a_0^{-1}$, alors détermination du stade préliminaire à l'état de transition de la réaction chimique partant de la géométrie de départ optimisée par un procédé comprenant les étapes suivantes :

> C détermination du stade préliminaire à l'état de transition de la réaction chimique
>
> C1 variation de la géométrie de départ optimisée à l'aide d'un algorithme de Monte-Carlo
>
> C1.1 au moins un atome de l'espace fonctionnel sélectionné dans l'étape B1 étant sélectionné aléatoirement,
>
> C1.2 un vecteur pour une déviation de l'atome sélectionné dans l'étape C1 étant sélectionné aléatoirement, la longueur sélectionnée aléatoirement du vecteur étant pondérée par la norme de gradient B3,
>
> C1.3 l'atome sélectionné dans l'étape C1.1 étant dévié à l'aide du vecteur de l'étape C1.2 à partir de la position de l'atome dans la géométrie de départ optimisée de telle sorte qu'un stade préliminaire à un état de transition de la réaction chimique est obtenu,
>
> C2 optimisation géométrique du stade préliminaire à l'état de transition au moyen de la méthode de chimie quantique et sous la condition limite selon laquelle ladite au moins une liaison de l'étape A2 présente la longueur qui a été déterminée dans l'étape C1.3,
>
> C3 détermination de la norme de gradient C3 pour le stade préliminaire à l'état de transition de l'étape C2, la norme de gradient étant obtenue par la première dérivée d'une fonction $E = f(x)$ de la méthode de chimie quantique, avec $E$ = énergie totale du stade préliminaire à l'état de transition et $x$ = les coordonnées de noyau de la molécule dans le stade préliminaire à l'état de transition,
>
> C4.1 lorsque la norme de gradient C3 V répond à $0 \leq \nabla \leq 0{,}03\ E_h\ a_0^{-1}$, alors poursuite du procédé par l'étape D1 ou
>
> C4.2 lorsque la norme de gradient C3 V répond à $> 0{,}03\ E_h\ a_0^{-1}\ E_h\ a_0^{-1}$, alors répétition des étapes C1 à C3 jusqu'à ce qu'une norme de gradient C3 V répondant à $0 \leq \nabla \leq 0{,}03\ E_h\ a_0^{-1}$ soit obtenue
>
>> (a) pour le cas où les étapes C1 à C3 ont été effectuées une fois, dans l'étape C1, la géométrie du stade préliminaire à l'état de transition étant modifiée lorsque la valeur de sa norme de gradient C3 est inférieure à la valeur de la norme de gradient B3 de la géométrie de départ optimisée ou
>>
>> (b) pour le cas où les étapes C1 à C3 ont été effectuées plus d'une fois, dans l'étape C1, la géométrie de la géométrie de départ optimisée ou du stade préliminaire respectif à l'état de transition, provenant des répétitions précédentes, qui présente la valeur la plus basse pour la norme de gradient C3 ou B3 par rapport à toutes les normes de gradient C3 et B3 obtenues jusqu'à présent, étant modifiée,
>
> D détermination de l'état de transition
>
> D1 variation du stade préliminaire de l'étape C4.1 ou du stade préliminaire de l'étape B4.1, en ce que les étapes suivantes pour un atome dans l'espace fonctionnel défini dans B1 sont effectuées
>
> D1.1 sélection d'un atome de l'espace fonctionnel sélectionné dans l'étape B1,
>
> D1.2 sélection d'un vecteur pour une déviation de l'atome sélectionné dans l'étape D1.1,
>
> D1.3 déviation de l'atome sélectionné dans l'étape D1.1 de sa position dans le stade préliminaire à l'aide du vecteur de l'étape D1.2, la position étant déviée une fois d'une valeur positive de la longueur du vecteur et une fois d'une valeur négative de la longueur du vecteur, de telle sorte que lorsque les étapes D1.1 à D.1.3 sont effectuées, on obtient deux stades préliminaires déviés,
>
> D2 optimisation géométrique des deux stades préliminaires déviés de l'étape D1 au moyen de la méthode de chimie quantique sous la condition limite selon laquelle les distances de liaison obtenues dans D1 sont maintenues constantes, de telle sorte que deux stades préliminaires optimisés (i) et (ii) sont obtenus,
>
> D3 calcul de l'énergie des deux stades préliminaires optimisés (i) et (ii) de l'étape D2 au moyen de la méthode de chimie quantique,

D4 comparaison de la valeur énergétique des deux stades préliminaires optimisés à la valeur énergétique C3 ou B3 du stade préliminaire qui a été utilisé dans l'étape D1,

D4.1. lorsque la valeur énergétique du stade préliminaire optimisé (i) et la valeur énergétique du stade préliminaire optimisé (ii) sont respectivement inférieures à la valeur énergétique C3 ou B3 du stade préliminaire qui a été utilisé dans l'étape D1, alors classification du stade préliminaire qui a été utilisé dans l'étape D1 comme état de transition,

D4.2. lorsque la valeur énergétique du stade préliminaire optimisé (i) ou la valeur énergétique du stade préliminaire optimisé (ii) ne sont pas respectivement inférieures à la valeur énergétique C3 ou

B3 du stade préliminaire qui a été utilisé dans l'étape D1, alors répétition du procédé à partir de l'étape C1.

13. Support d'enregistrement lisible par un ordinateur, comprenant des instructions qui permettent de mettre en œuvre les étapes suivantes d'un procédé lors de l'exécution par un ordinateur

A génération d'une géométrie de départ

A1 mise à disposition de la représentation tridimensionnelle d'au moins une molécule dans l'état énergétique de base,

A2 sélection d'au moins une liaison de ladite au moins une molécule et sélection d'une longueur de la liaison, la longueur sélectionnée ne correspondant pas à la longueur de la liaison dans l'état énergétique de base de la molécule, de telle sorte qu'une géométrie de départ pour la réaction chimique est obtenue,

A3 représentation tridimensionnelle de la géométrie de départ en coordonnées cartésiennes et/ou internes,

B détermination d'une géométrie de départ optimisée

B1 définition d'un espace fonctionnel qui comprend ladite au moins une liaison de l'étape A2 et les atomes reliés par cette liaison,

B2 optimisation géométrique de l'espace fonctionnel sélectionné dans l'étape B1 au moyen d'une méthode de chimie quantique et sous la condition limite selon laquelle la longueur sélectionnée dans l'étape A2 de ladite au moins une liaison est maintenue constante, de telle sorte qu'une géométrie de départ optimisée est obtenue,

B3 détermination de la norme de gradient B3 pour la géométrie de départ optimisée, la norme de gradient étant obtenue par la première dérivée d'une fonction $E = f(x)$ de la méthode de chimie quantique, avec $E$ = énergie totale de la géométrie de départ optimisée et $x$ = les coordonnées de noyau de la molécule dans la géométrie de départ optimisée,

B4.1 lorsque la norme de gradient B3 V répond à $0 \leq \nabla \leq 0,03\ E_h\ a_0^{-1}$, alors classification de la géométrie de départ optimisée comme un stade préliminaire à l'état de transition de la réaction chimique et poursuite du procédé par l'étape D1 ou

B4.2 lorsque la norme de gradient B3 répond à $V > 0,03\ E_h\ a_0^{-1}$, alors détermination du stade préliminaire à l'état de transition de la réaction chimique partant de la géométrie de départ optimisée par un procédé comprenant les étapes suivantes :

C détermination du stade préliminaire à l'état de transition de la réaction chimique

C1 variation de la géométrie de départ optimisée à l'aide d'un algorithme de Monte-Carlo

C1.1 au moins un atome de l'espace fonctionnel sélectionné dans l'étape B1 étant sélectionné aléatoirement,

C1.2 un vecteur pour une déviation de l'atome sélectionné dans l'étape C1 étant sélectionné aléatoirement, la longueur sélectionnée aléatoirement du vecteur étant pondérée par la norme de gradient B3,

C1.3 l'atome sélectionné dans l'étape C1.1 étant dévié à l'aide du vecteur de l'étape C1.2 à partir de la position de l'atome dans la géométrie de départ optimisée de telle sorte qu'un stade préliminaire à un état de transition de la réaction chimique est obtenu,

C2 optimisation géométrique du stade préliminaire à l'état de transition au moyen de la méthode de chimie quantique et sous la condition limite selon laquelle ladite au moins une liaison de l'étape A2 présente la longueur qui a été déterminée dans l'étape C1.3,

C3 détermination de la norme de gradient C3 pour le stade préliminaire à l'état de transition de l'étape C2, la norme de gradient étant obtenue par la première dérivée d'une fonction $E = f(x)$ de la méthode de chimie quantique, avec $E$ = énergie totale du stade préliminaire à l'état de transition et $x$ = les coordonnées de noyau de la molécule dans le stade préliminaire à l'état de transition,

C4.1 lorsque la norme de gradient C3 V répond à $0 \leq \nabla \leq 0,03\ E_h\ a_0^{-1}$, alors poursuite du procédé par l'étape D1 ou

C4.2 lorsque la norme de gradient C3 V répond à $> 0,03\ E_h\ a_0^{-1}\ E_h\ a_0^{-1}$, alors répétition des étapes C1 à C3 jusqu'à ce qu'une norme de gradient C3 V répondant à $0 \leq \nabla \leq 0,03\ E_h\ a_0^{-1}$ soit obtenue

(a) pour le cas où les étapes C1 à C3 ont été effectuées une fois, dans l'étape C1, la géométrie du stade préliminaire à l'état de transition étant modifiée lorsque la valeur de sa norme de gradient C3 est inférieure à la valeur de la norme de gradient B3 de la géométrie de départ optimisée ou

(b) pour le cas où les étapes C1 à C3 ont été effectuées plus d'une fois, dans l'étape C1, la géométrie de la géométrie de départ optimisée ou du stade préliminaire respectif à l'état de transition, provenant des répétitions précédentes, qui présente la valeur la plus basse pour la norme de gradient C3 ou B3 par rapport à toutes les normes de gradient C3 et B3 obtenues jusqu'à présent, étant modifiée,

D détermination de l'état de transition

D1 variation du stade préliminaire de l'étape C4.1 ou du stade préliminaire de l'étape B4.1, en ce que les étapes suivantes pour un atome dans l'espace fonctionnel défini dans B1 sont effectuées

D1.1 sélection d'un atome de l'espace fonctionnel sélectionné dans l'étape B1,

D1.2 sélection d'un vecteur pour une déviation de l'atome sélectionné dans l'étape D1.1,

D1.3 déviation de l'atome sélectionné dans l'étape D1.1 de sa position dans le stade préliminaire à l'aide du vecteur de l'étape D1.2, la position étant déviée une fois d'une valeur positive de la longueur du vecteur et une fois d'une valeur négative de la longueur du vecteur, de telle sorte que lorsque les étapes D1.1 à D.1.3 sont effectuées, on obtient deux stades préliminaires déviés,

D2 optimisation géométrique des deux stades préliminaires déviés de l'étape D1 au moyen de la méthode de chimie quantique sous la condition limite selon laquelle les distances de liaison obtenues dans D1 sont maintenues constantes, de telle sorte que deux stades préliminaires optimisés (i) et (ii) sont obtenus,

D3 calcul de l'énergie des deux stades préliminaires optimisés (i) et (ii) de l'étape D2 au moyen de la méthode de chimie quantique,

D4 comparaison de la valeur énergétique des deux stades préliminaires optimisés à la valeur énergétique C3 ou B3 du stade préliminaire qui a été utilisé dans l'étape D1,

D4.1. lorsque la valeur énergétique du stade préliminaire optimisé (i) et la valeur énergétique du stade préliminaire optimisé (ii) sont respectivement inférieures à la valeur énergétique C3 ou B3 du stade préliminaire qui a été utilisé dans l'étape D1, alors classification du stade préliminaire qui a été utilisé dans l'étape D1 comme état de transition,

D4.2. lorsque la valeur énergétique du stade préliminaire optimisé (i) ou la valeur énergétique du stade préliminaire optimisé (ii) ne sont pas respectivement inférieures à la valeur énergétique C3 ou B3 du stade préliminaire qui a été utilisé dans l'étape D1, alors répétition du procédé à partir de l'étape C1.

14. Utilisation d'un programme informatique selon la revendication 12 ou d'un support d'enregistrement lisible par un ordinateur selon la revendication 13 pour l'évaluation d'états de transition d'une réaction chimique, en particulier d'une synthèse de polymères.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **LIN et al.** A flexible transition state searching method for atmospheric reaction systems. *Chemical Physics*, 2015, vol. 450-451, 21-31
- **E. MARTÍNEZ-NÚÑEZ et al.** An automated transition state search using classical trajectories initialized at multiple minima. *Phys Chem Chem Phys*, 14 June 2015, vol. 17 (22), 14912-21
- tsscds2018: A code for automated discovery of chemical reaction mechanisms and solving the kinetics. *J. Comp.Chem.*, 24 September 2018, vol. 39 (23), 1922-1930
- **JACOBSON et al.** Automated Transition State Search and Its Application to Diverse Types of Organic Reactions. *J. Chem. Theory Comput.*, vol. 13 (11), 5780-5797
- **HU et al.** A gradient-directed Monte Carlo method for global optimization in a discrete space: Application to protein sequence design and folding. *J Chem Phys.*, 21 October 2009, vol. 131 (15), 154117